# FASCICULE DE BREVET EUROPEEN

(11) **EP 2 935 281 B1**
(45) Date de publication et mention de la délivrance du brevet: **09.09.2020**
(21) Numéro de dépôt: 13814940.6
(22) Date de dépôt: 20.12.2013
(51) Int. Cl.: C07D 498/10, A61K 31/435, A61P 31/04

(54) **COMPOSES DE TYPE SPIROISOXAZOLINE AYANT UNE ACTIVITE POTENTIALISATRICE DE L'ACTIVITE D'UN ANTIBIOTIQUE**
SPIROISOXAZOLINVERBINDUNGEN ZUR AKTIVITÄTSPOTENZIERUNG EINES ANTIBIOTIKUMS
SPIROISOXAZOLINE COMPOUNDS HAVING AN ACTIVITY POTENTIATING THE ACTIVITY OF AN ANTIBIOTIC

(30) Priorité: 21.12.2012 FR 1203549
(43) Date de publication de la demande: 28.10.2015
(73) Titulaire: Université de Droit et de la Santé de Lille 2, 59800 Lille (FR)
(72) Inventeur: WILLAND, Nicolas, F-59000 Lille (FR); DEPREZ, Benoit, F-59000 Lille (FR); BAULARD, Alain, B-7520 Tournai (BE); BRODIN, Priscille, F-75014 Paris (FR); DESROSES, Matthieu Frédérik, S-74143 Knivsta (SE); AGOURIDAS-DUTOT, Laurence, F-59800 Lille (FR)
(74) Mandataire: Latscha Schöllhorn Partner AG
(86) Numéro de dépôt international: PCT/EP2013/077706
(87) Numéro de publication internationale: WO 2014/096369

(56) Documents cités:
- WO-A1-2006/122770
- MARION FLIPO ET AL: "Ethionamide Boosters: Synthesis, Biological Activity, and Structure-Activity Relationships of a Series of 1,2,4-Oxadiazole EthR Inhibitors", JOURNAL OF MEDICINAL CHEMISTRY, vol. 54, no. 8, 28 avril 2011 (2011-04-28) , pages 2994-3010, XP055019892, ISSN: 0022-2623, DOI: 10.1021/jm200076a cité dans la demande
- BAULARD A R ET AL: "Activation of the pro-drug ethionamide is regulated in mycobacteria", JOURNAL OF BIOLOGICAL CHEMISTRY, AMERICAN SOCIETY FOR BIOCHEMISTRY AND MOLECULAR BIOLOGY, US, vol. 275, no. 36, 8 septembre 2000 (2000-09-08), pages 28326-28331, XP002283429, ISSN: 0021-9258 cité dans la demande

## Description

La présente invention concerne des composés de type spiroisoxazoline pour leur utilisation dans le traitement des infections bactériennes et mycobactériennes comme, par exemple, la tuberculose, la lèpre et les infections mycobactériennes atypiques.

La présente invention concerne également de nouveaux composés utilisables en tant que médicament en particulier en tant que médicament pour le traitement d'infections bactériennes et mycobactériennes comme, par exemple, la tuberculose, la lèpre et les infections mycobactériennes atypiques.

La présente invention concerne également des compositions pharmaceutiques comprenant, en tant que principe actif, au moins un des composés précités et éventuellement un antibiotique actif contre les bactéries et/ou mycobactéries, notamment un antibiotique activable par la voie de l'EthA.

La présente invention concerne également des produits (kits) contenant au moins un des composés précités et au moins un antibiotique actif contre les bactéries et/ou mycobactéries, notamment un antibiotique activable selon la voie de l'EthA comme produits de combinaison pour une utilisation simultanée, séparée ou étalée dans le temps en thérapie antituberculeuse, antilépromateuse ou antimycobactérienne générale.

La tuberculose tue 2 millions de personnes chaque année dans le monde. L'épidémie de sida et l'émergence de souches multi résistantes aux antibiotiques contribue à aggraver l'impact de cette maladie, considérée par l'Organisation Mondiale de la Santé comme responsable d'une épidémie mondiale de plus en plus dangereuse et comme une urgence sanitaire au niveau planétaire.

Un nombre croissant de souches de *Mycobacterium tuberculosis* est aujourd'hui caractérisé par une multi résistance aux antibiotiques de première ligne que sont l'isoniazide (INH) et la rifampicine (RIF). Ces antibiotiques doivent alors être remplacés par des antibiotiques de seconde ligne comme l'éthionamide (ETH) auxquels les souches ne sont pas résistantes mais qui ont le désavantage de présenter un indice thérapeutique faible (l'indice thérapeutique d'un principe actif est le rapport de la dose thérapeutique à la dose toxique).

Une stratégie consistant à augmenter l'activité de l'éthionamide (ETH) en l'associant à un composé particulier a déjà été envisagée. En effet l'ETH est une prodrogue qui est transformée *in vivo* en une forme thérapeutiquement active par l'enzyme EthA (voir l'article "Activation of the prodrug ethionamide is regulated in mycobacteria", A.R. Baulard et al., Journal of Biological Chemistry, 2000, 275, 28326-28331). Les résistances observées à l'ETH viennent du fait que le répresseur transcriptionnel EthR de *M*. *tuberculosis* contrôle l'expression de l'enzyme EthA et limite la transformation de l'ETH en substance thérapeutiquement active.

Un but de la présente invention est de proposer de nouveaux composés susceptibles de potentialiser l'activité des antibiotiques actifs notamment contre les mycobactéries, en particulier les antibiotiques actifs contre les mycobactéries et activables par la voie EthA, comme, par exemple, tous les antibiotiques de la famille des thioamides et en particulier, l'éthionamide et le prothionamide.

Un autre but de la présente invention est de proposer des composés tels que précités qui permettent en combinaison avec un antibiotique actif contre les bactéries et/ou mycobactéries, notamment un antibiotique actif contre les mycobactéries et activable par la voie de l'EthA, d'obtenir à dose d'antibiotique égale une meilleure efficacité ou qui permettent de réduire la dose d'antibiotique précitée pour obtenir une efficacité donnée.

Un autre but de la présente invention est de proposer des composés tels que précités qui soient simples et peu coûteux à produire.

Un autre but de la présente invention est de proposer des composés tels que précités qui soient solubles de manière satisfaisante dans un fluide biologique.

Un autre but de la présente invention est de proposer des composés tels que précités qui sont susceptibles d'être actifs en particulier par voie orale et/ou qui engendrent moins d'effets secondaires.

Pour atteindre au moins un des objectifs précités, la présente invention propose donc des composés de formule générale (I) : 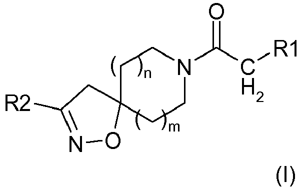 dans laquelle :
m = 0 ou 1 ;
n = 0 ou 1 ;
R1 représente un groupement choisi parmi les groupements suivants :
   les chaînes alkyles en C1-C5, linéaires ou ramifiées ;
   les chaînes alkyles en C1-C5 linéaires ou ramifiées et substituées, en particulier substituées par au moins un atome de fluor (F),
   en particulier les chaînes alkyles en C1-C3 linéaires ou ramifiées et substituées par au moins un atome de fluor (F) ou par un groupement cyclique saturé ou insaturé en C3-C6 ;
   les groupements cycliques saturés ou insaturés en C3-C6 ; et
   les groupements CN, CH₂CN, CH₂N₃ ;
R2 est choisi parmi les groupements suivants :
   phényle ;
   benzyle éventuellement substitué, en particulier benzyle substitué par un atome Cl ou F ; naphatalènyle ;
   phényle substitué, en particulier phényle substitué par au moins une chaîne alkyle en C1-C4, linéaire ou ramifiée ;
   phényle substitué par au moins une chaîne alkyle en C1-C4 linéaire ou ramifiée et substituée, en particulier substituée par au moins un atome de fluor (F) ;
   phényle substitué par au moins un groupement choisi parmi OCH₃, OCF₃, Cl, F, CH₃SO₂ et CF₃ ;
   un groupement phényle dont deux atomes de carbone consécutifs sont substitués par un même groupement O-CH₂-O ;
   un groupement cyclopropyle, cyclobutyle, cyclopentyle, ou cyclohexyle ; et
   les hétérocycles à 5 ou 6 sommets saturés ou insaturés comportant au moins un atome dans le cycle choisi parmi S, N et O, en particulier les hétérocyles aromatiques à 5 ou 6 sommets comportant au moins un atome dans le cycle choisi parmi S, N et O.

Selon l'invention m et n peuvent être égaux ou différents l'un de l'autre. Avantageusement, m = n = 1. De tels composés s'avèrent être particulièrement actifs en combinaison avec l'éthionamide sur les mycobactéries, en particulier sur *M*. *tuberculosis.*

R1 peut représenter un groupement choisi parmi les groupements suivants :
-CH₂CF₃, -CF₂CF₃, -(CH₂)₂CF₃, -CH₂-isopropyle, cyclopropyle, cyclobutyle, cyclopentyle, cyclopentènyle, -CH₂-cyclopropyle, -CH₂-cyclobutyle, -CH₂-cyclopentyle, phényle éventuellement substitué, en particulier phényle substitué par un atome de chlore (Cl) ou de fluor (F), benzyle éventuellement substitué, en particulier benzyle substitué par un atome Cl ou F, -O-phényle et thiophènyle.

Les groupements R1 substitués précités peuvent être monosubstitués ou pluri-substitués. Selon un mode de réalisation, les groupements précités sont monosubstitués ; le groupe phényle ou benzyle est alors substitué uniquement par un atome Cl ou un atome F. La position de cet atome Cl ou F n'est pas limitée selon l'invention, que le groupement phényle ou benzyle soit monosubstitué ou pluri-substitué. Ainsi, l'atome Cl ou F peut être en position ortho, méta ou para par rapport au carbone du cycle benzénique lié au reste de la molécule de formule générale (I) ou par rapport au carbone du cycle benzénique lié au groupement CH₂ lié au reste de la molécule de formule générale (I), dans le cas d'un groupement benzyle.

Avantageusement, R1 est choisi parmi -CH₂CF₃ et -(CH₂)₂CF₃. Ces deux radicaux procurent aux composés de l'invention une efficacité améliorée. Il semble que, en particulier lorsque R1= CH₂CF₃, le composé de l'invention est particulièrement efficace pour augmenter l'activité de l'éthionamide sur les mycobactéries et en particulier sur M. *tuberculosis.*

Selon un mode de réalisation, R2 est choisi parmi les groupements suivants : phényle, les groupements phényles substitués en ortho par OCH₃, OCF₃, Cl, F ou CF₃, en particulier substitué en ortho par OCH₃ ou Cl. Avantageusement, les groupements phényles sont monosubstitués en ortho par un radical choisi parmi OCH₃, OCF₃, Cl, F ou CF₃, en particulier monosubstitué en ortho par OCH₃ ou Cl. De tels radicaux améliorent l'activité potentialisatrice de l'éthionamide des composés de l'invention. La position en ortho du substituant améliore l'activité potentialisatrice de l'éthionamide des composés de l'invention.

Selon un autre mode de réalisation, R2 est choisi parmi les hétérocycles aromatiques à 5 ou 6 atomes dont au moins un atome adjacent à l'atome de l'hétérocycle lié en position alpha par rapport à l'azote du cycle 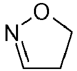 du composé de formule générale (I) de l'invention est un atome de soufre, un atome d'oxygène ou un atome d'azote. Selon l'invention, les hétérocycles à 5 ou 6 atomes peuvent donc comporter deux atomes choisis parmi les atomes S, N et O. Dans ce cas, les deux atomes choisis parmi S, N et O peuvent être situés de part et d'autre de l'atome de l'hétérocycle lié au cycle au carbone en alpha de l'azote du cycle 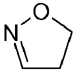 dans la formule (I) et directement adjacent à ce dernier.

L'atome de l'hétérocycle lié au cycle précité est avantageusement un atome de carbone.

Avantageusement, R2 est choisi parmi les groupements suivants

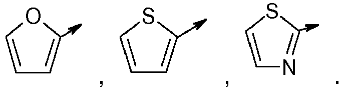

De tels composés sont particulièrement efficaces en combinaison avec l'éthionamide pour lutter contre les mycobactéries, en particulier *M*. *tuberculosis.*

Selon un autre mode de réalisation, R2 est choisi parmi les groupements suivants :

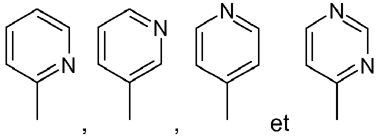

Le composé de l'invention est avantageusement choisi parmi les composés suivants :

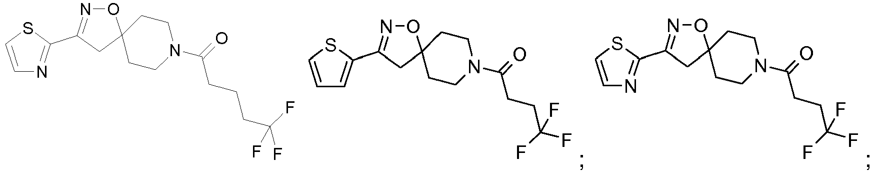

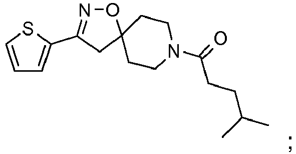

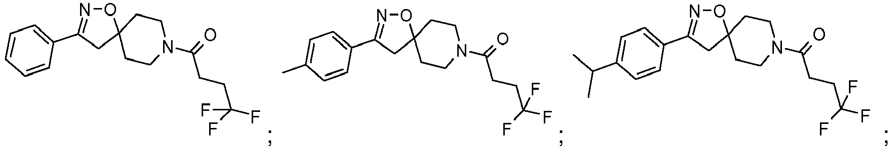

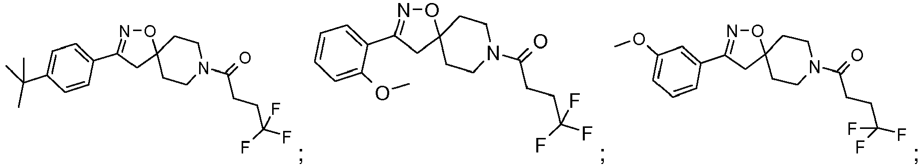

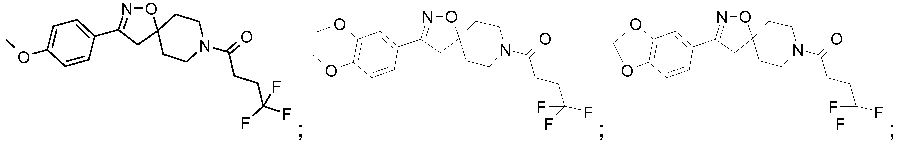

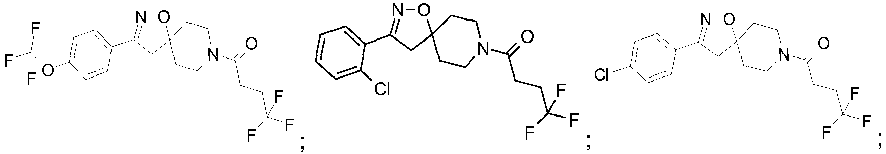

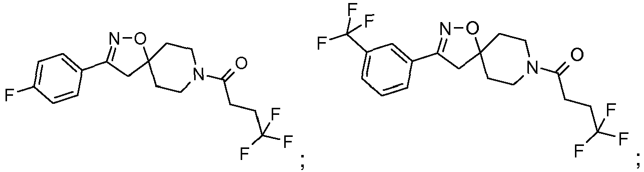

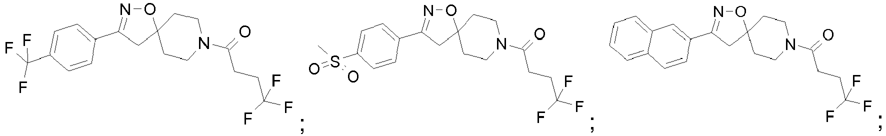

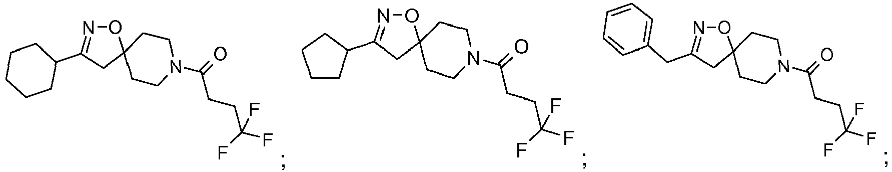

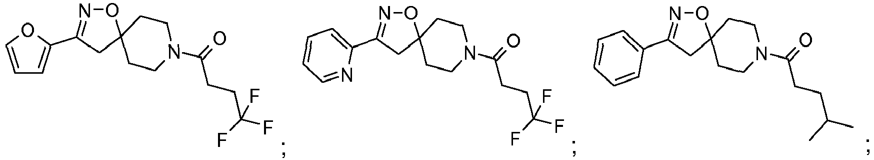

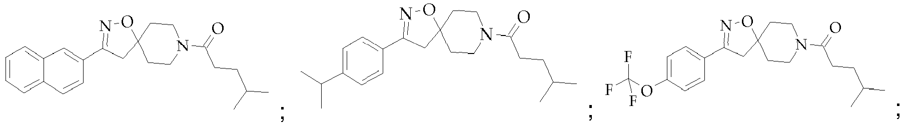

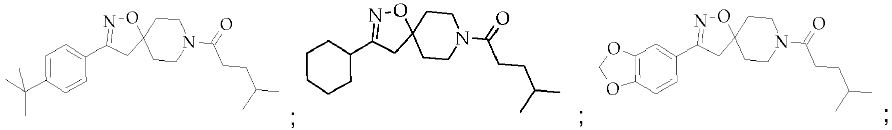

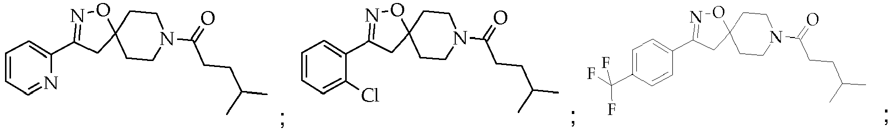

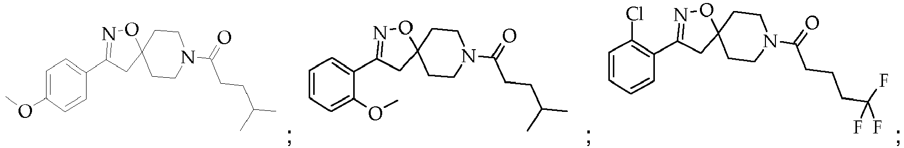

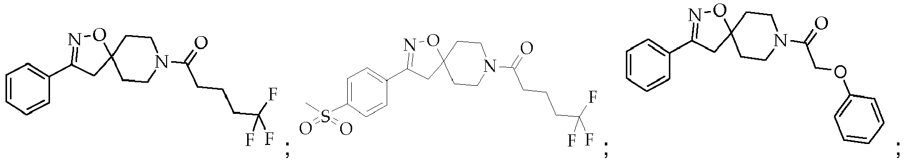

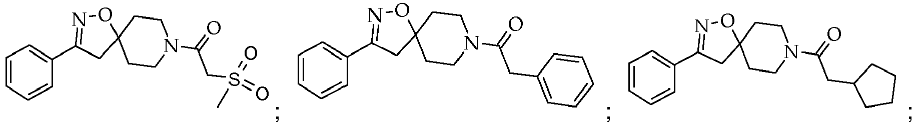

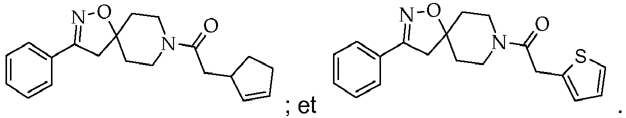

La présente invention concerne également les composés précités pour leur utilisation en tant que médicament, en particulier, pour leur utilisation dans le traitement des infections bactériennes et mycobactériennes, notamment dans le traitement de la tuberculose, de la lèpre ou de mycobactérioses atypiques.

La présente invention concerne également une composition pharmaceutique comprenant, en tant que principe actif, au moins un composé de formule générale (I) telle que précitée et un excipient pharmaceuticalement acceptable.

Au sein des compositions pharmaceutiques conformes à l'invention, le ou les composés utilisés en tant qu'ingrédient(s) actif(s) peuvent être utilisés en une quantité permettant d'administrer des doses unitaires comprises entre 0,3 mg et 1 g environ. Au sein des compositions pharmaceutiques conformes à l'invention et lorsqu'il(s) est(sont) présent(s), le ou les antibiotiques activables par la voie enzymatique de l'EthA sont avantageusement utilisés en une quantité permettant d'administrer des doses unitaires égales ou inférieures aux doses habituellement recommandées par l'OMS (WHO, Treatment of tuberculosis: Guidelines for National Programmes. 2003; WHO/CDS/ TB2003.313.), les organisations sanitaires nationales ou non gouvernementales, ou les laboratoires pharmaceutiques compétents.

L'Homme du Métier est à même de choisir un ou plusieurs excipients pharmaceutiquement acceptables en fonction de la voie d'administration de la composition pharmaceutique. Bien entendu, l'Homme du Métier veillera à cette occasion à ce que le ou les excipients utilisés soient compatibles avec les propriétés intrinsèques attachées à la composition conforme à la présente invention. En outre, la forme du médicament ou de la composition pharmaceutique (par exemple, une solution, une suspension, une émulsion, des comprimés, des gélules, des suppositoires, etc...) dépendra de la voie d'administration choisie.

Ainsi, au sens de la présente Invention, le médicament ou la composition pharmaceutique peut être administré(e) par n'importe quelle voie appropriée, par exemple par la voie orale, anale, locale (topique, par exemple), systémique, intraveineuse, intramusculaire ou mucosale, ou bien en utilisant un patch, ou encore sous forme encapsulée dans, ou immobilisée sur, des liposomes, des microparticules, des microcapsules, associée à des nanoparticules et analogues. On peut notamment citer, à titre d'exemples non limitatifs d'excipients appropriés pour une administration par voie orale, le talc, le lactose, l'amidon et ses dérivés, la cellulose et ses dérivés, les polyéthylèneglycols, les polymères d'acide acrylique, la gélatine, le stéarate de magnésium, des matières grasses animales, végétales ou synthétiques, les dérivés de la paraffine, les glycols, les stabilisants, les conservateurs, les anti-oxydants, les agents mouillants, les anti-agglomérants, les dispersants, les émulsionnants, les agents modifiants du goût, les agents de pénétrations, de solubilisation, etc.... Les techniques de formulation et d'administration des médicaments et compositions pharmaceutiques sont bien connues dans la technique ici considérée, l'Homme du Métier pouvant notamment se référer à l'ouvrage Remington's Pharmaceutical Sciences, dernière édition.

La présente Invention a également pour objet l'utilisation d'au moins un composé selon l'invention pour la fabrication d'un médicament destiné à la prévention et/ou au traitement des infections bactériennes, de préférence mycobactériennes, et tout particulièrement de la tuberculose, de la lèpre ou de mycobactérioses atypiques.

Avantageusement, la composition pharmaceutique comprend en outre, en tant que principe actif, au moins un antibiotique actif contre les bactéries et/ou mycobactéries, choisi notamment parmi les antibiotiques activables par la voie enzymatique de l'EthA, en particulier parmi l''éthionamide, le prothionamide, l'isoxyl et le thiacétazone qui sont des exemples d'antibiotiques activables par la voie EthA. La présente invention n'est néanmoins pas limitée à ces antibiotiques.

La présente invention concerne également un kit ou produit contenant au moins un composé de formule (I) et au moins un antibiotique actif contre les bactéries et/ou mycobactéries choisi, notamment, parmi les antibiotiques activables par la voie enzymatique de l'EthA, comme produits de combinaison pour une utilisation simultanée, séparée ou étalée dans le temps en thérapie antituberculeuse, anti-lépromateuse ou antimycobactérienne générale.

### DEFINITIONS

On définit au sens de la présente invention le groupement thiophènyle comme un groupement cyclique comportant 4 atomes de carbone et un atome de soufre.

Lorsqu'il n'est pas précisé qu'un groupement est substitué, celui-ci n'est pas substitué, au sens de la présente invention.

Lorsque la position du substituant n'est pas précisée, le terme phényle substitué concerne tout groupement phényle dont au moins un atome d'hydrogène est remplacé par un groupement ou un atome tel qu'indiqué. De préférence, pour R2, le groupement phényle est monosubstitué, de préférence monosubstitué par un groupement OCH₃, un atome de chlore ou un atome de fluor. Il peut néanmoins comprendre au moins deux substituants.

La position ortho du substituant porté par le phényle fait référence à l'atome de carbone du groupement phényle qui est lié à l'atome de carbone en position alpha par rapport à l'azote du cycle 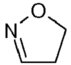 des composés de l'invention.

Les hétérocycles à 5 ou 6 atomes peuvent saturés ou insaturés. Ils peuvent comprendre une ou plusieurs double-liaison. Ils peuvent également être des cycles aromatiques. Ainsi, au sens de la présente invention, les termes hétérocycles aromatiques à 5 ou 6 sommets comportant au moins un atome choisi parmi N, O et S dans le cycle englobent, notamment, les hétérocycles aromatiques à 5 ou 6 sommets comportant au moins un atome d'azote dans le cycle et uniquement un ou des atomes N en tant qu'hétéroatome dans le cycle, les hétérocycles aromatiques à 5 ou 6 sommets, en particulier à 5 sommets, comportant au moins un atome O dans le cycle et uniquement un ou des atomes O en tant qu'hétéroatome(s) dans le cycle, les hétérocycles aromatiques à 5 ou 6 sommets comportant au moins un atome S dans le cycle et uniquement un ou des atomes S en tant qu'hétéroatome(s) dans le cycle, les hétérocycles aromatiques à 5 ou 6 sommets, en particulier à 5 sommets, comportant au moins un atome d'azote dans le cycle et/ou au moins un atome O ou S dans le cycle en tant qu'hétéroatomes, En particulier, les hétérocycles aromatiques à 5 ou 6 sommets comportant deux hétéroatomes différents ou identiques choisis parmi S, N et O font partie des hétérocycles aromatiques à 5 ou 6 sommets selon l'invention.

On définit ici les mycobactérioses atypiques comme étant les mycobactérioses causées par au moins une mycobactérie autre que *M. Tuberculinum* et en particulier les mycobactérioses impliquant *M*. *Kansasii.*

Au sens de la présente invention, le terme « traitement » désigne le traitement curatif et/ou le traitement prophylactique des infections précitées. Le terme « traitement » englobe toute amélioration de l'état du patient, en particulier toute diminution de la quantité de bactéries présentes dans un moins un site d'infection du patient.

On définit au sens de la présente invention, un antibiotique activable par la voie de l'EthA, toute substance qui, au moins *in vitro* réagit avec l'enzyme EthA pour produire une substance ayant des propriétés antibiotiques. L'Homme du métier est à même de déterminer si un antibiotique est activable par la voie de l'EthA, par exemple, en appliquant la méthode décrite dans la publication suivante : "Activation of the prodrug ethionamide is regulated in mycobacteria", A.R. Baulard et al., Journal of Biological Chemistry, 2000, 275, 28326-28331.

On définit au sens de la présente invention, un antibiotique actif contre les bactéries et/ou mycobactéries, tout agent susceptible de limiter ou de réduire au moins *in vitro* la prolifération d'une bactérie et/ou d'une mycobactérie, en particulier *M*. *tuberculosis.* Un agent susceptible de détruire, au moins in vitro une mycobactérie, notamment M. *tuberculosis* est également un antibiotique actif contre les mycobactéries, au sens de la présente invention. Parmi les antibiotiques actifs contre les mycobactéries et activables par la voie de l'EthA, on peut citer l'éthionamide, le prothionamide, l'isoxyl, le thiacétazone et les mélanges d'au moins deux de ces antibiotiques.

L'antibiotique au sens la présente invention peut aussi être un antibiotique activable par une autre voie de bioactivation que celle précitée.

### PARTIE EXPERIMENTALE

### SYNTHESES

Les spectres RMN ¹H et ¹³C ont été réalisés à température ambiante sur un appareil Bruker™ DPX 300 à 300 MHz. Les déplacements chimiques sont indiqués en partie par million (ppm). Les assignements ont été réalisés en utilisant les expériences à une dimension (1D) ¹H et ¹³C ou à deux dimensions (2D) HSQC et COSY. Les spectres de masse ont été réalisés sur une LCMS-MS équipée d'un système triple quadripole (Varian 1200ws) ou sur une LCMS Waters Alliance Micromass ZQ 2000. Les réactifs et solvants commerciaux ont été utilisés sans purification ultérieure.

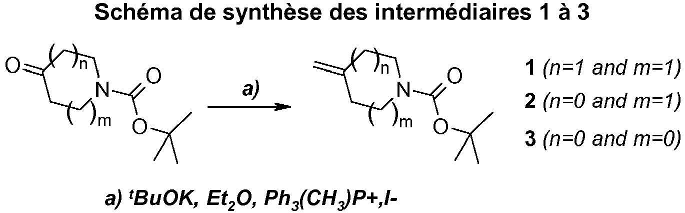

**Synthèse des intermédiaires 1, 2 et 3.** Le *tert*-butoxyde de potassium (1.5 mmoles) est additionné à la suspension sous argon, d'iodure de triphénylméthylphosphonium (1.5 mmoles) dans l'éther anhydre (2.0 mL). Le mélange est porté à reflux durant 1h et vire alors au jaune. Le composé cétonique est ensuite additionné par portions. Après 30 minutes d'agitation à température ambiante, l'analyse par CCM indique que la réaction est achevée. Le milieu réactionnel est alors hydrolysé à l'aide d'eau distillée (1 mL), et extrait par deux fois à l'aide d'éther. Les phases organiques jointes sont séchées sur MgSO₄ et concentrées à sec. Le résidu obtenu est purifié sur colonne de gel de silice (éluant cyclohexane / acétate d'éthyle) pour donner le produit attendu sous forme d'huile incolore.

**1-N-Boc-4-methylenepiperidine (1)**. NMR ¹H (CDCl₃, 300 MHz) δ(ppm) 1.47 (s, 9H, CH₃ Boc), 2.18 (t, 4H, *J*=5.9 Hz, CH₂), 3.42 (t, 4H, *J*=5.8 Hz, CH₂N), 4.74 (s, 2H, =CH₂). 70% rendement. [(M-tBu),H]⁺ 142.1. Pureté >95%.

**1-N-Boc-3-methylenepyrrolidine (2)**. NMR ¹H (CDCl₃, 300 MHz) δ (ppm) 1.48 (s, 9H, CH₃ Boc), 2.55 (t, 2H, *J*=7.2 Hz, CH₂), 3.48 (m, 2H, CH₂), 3.93 (m, 2H, CH₂), 4.98 (m, 2H, =CH₂). 83% rendement. [(M-tBu),H]⁺ 128.1. Pureté >95%.

**1-N-Boc-3-methyleneazetidine (3)**. NMR ¹H (CDCl₃, 300 MHz) δ (ppm) 1.46 (s, 9H CH₃ Boc), 4.49 (t, 4H, *J*=2.7 Hz, CH₂), 4.99 (q, 2H, *J*=2.4 Hz, CH₂). 69% rendement. [(M-tBu),H]+ 114.3. Pureté >95%.

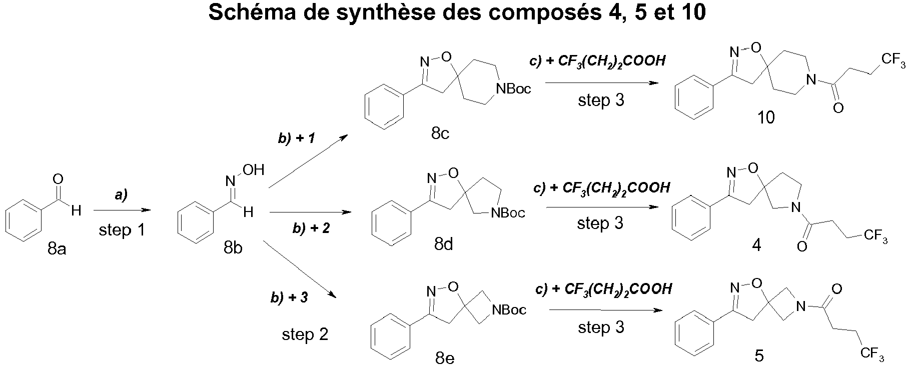

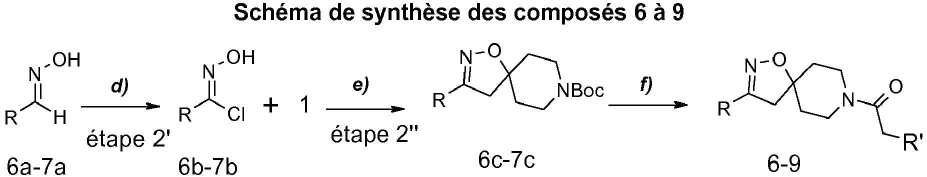

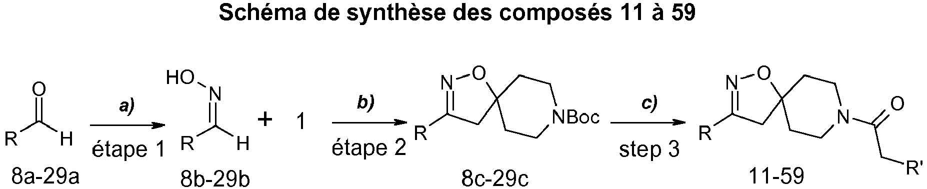

Conditions opératoires: ***(a)*** NH₂OH.HCl, CH₂Cl₂, pyridine, RT; ***(b)*** NaOCl 13%, CH₂Cl₂, 0°C to RT; ***(c)** (i)* HCl (4M) in 1,4-dioxane, *(ii)* R'-CO₂H, HOBt, EDCl.HCl, DIEA, DMF; ***(d)*** NCS, DMF, RT, ***(e)*** 1-N-Boc-4-methylene piperidine, Et₃N, THF, RT; ***(f)**(i)* HCl (4M) in 1,4-dioxane, *(ii)* CF₃(CH₂)₂-CO₂H, HOBt, EDCl.HCl, DIEA, DMF.

**Etape 1: synthèse des oximes:** Le chlorhydrate d'hydroxylamine (1 mmole) est additionné à la solution d'aldéhyde (1 mmole) dans le dichlorométhane (860 µL) et la pyridine (80 µL). Le mélange est ensuite agité à température ambiante. L'avancement et la fin de la réaction sont suivis par chromatographie sur couche mince (CCM). Du dichlorométhane est ajouté puis la phase organique est lavée à l'aide d'eau distillée, séchée sur MgSO₄ et concentrée à sec. L'oxime ainsi obtenue est utilisée sans nécessité de purification ultérieure.

**Etape 2: Procédure pour la synthèse des intermédiaires 8c à 29c**. La solution d'hypochlorite de sodium (solution 13% en chlore, 3.4 mL) est additionnée à la solution d'oxime (1 mmole) et d'alcène 1, 2 ou 3 (0.8 mmoles) dans le CH₂Cl₂ abaissée à 0°C. Le mélange est agité à température ambiante jusqu'à ce que la réaction soit achevée. Les deux phases sont séparées et la phase organique est alors successivement lavée à l'aide d'HCl 1N (2 fois), d'une solution saturée en NaHCO₃ et de saumure avant d'être évaporée sous pression réduite. Si nécessaire, le dérivé spiroisoxazoline obtenu est purifié par HPLC préparative ou sur chromatographie flash (éluant cyclohexane / acétate d'éthyle).

**Etape 2' et 2": Procédure pour la synthèse des dérivés 6b-6c et 7b-7c**. Le N-chloro-succinimide (1 mmole) est ajouté à la solution d'oxime (1 mmole) dans le DMF (1.9 mL). Après 1h d'agitation à température ambiante, (le contrôle LC/MS indiquant la fin de la reaction), le DMF est évaporé sous pression réduite. Le résidu obtenu est repris dans le CH₂Cl₂, puis est lavé par 2 fois à l'aide de saumure. La phase organique est séchée sur MgSO₄ et concentrée à sec. La solution de triéthylamine (2 mmoles) dans le THF (32 mL) est additionnée goutte à goutte, durant 4h, à la solution de chloro-oxime et de 1-N-Boc-4-méthylène-pipéridine (1.3 mmoles) dans le THF (32 mL). Le milieu réactionnel est agité à température ambiante. Le chlorhydrate de triéthylamine ainsi formé est éliminé par filtration et le THF est évaporé sous pression réduite. Le résidu obtenu est repris dans le dichlorométhane et est lavé successivement à l'aide d'une solution d'HCl 1N et de saumure. La phase organique est ensuite séchée sur MgSO₄ et concentrée à sec. Le résidu recueilli est purifié sur colonne de gel de silice (éluant CH₂Cl₂/EtOAc).

**Etape 3: déprotection du groupement N-Boc**. La solution d'HCl 4N dans le dioxane (2.2 mL) est additionnée à la solution de N-Boc-amine (1 mmole) dans le dioxane (4.4 mL). Le milieu réactionnel est agité à température ambiante. Une fois la réaction terminée (suivi par CCM), le précipité formé est essoré sur verre fritté et utilisé sans purification ultérieure dans l'étape de couplage.

**Couplage avec les acides carboxyliques**: La solution d'acide carboxylique (1.5 mmoles), d'EDCl.HCl (1.5 mmoles), d'HOBt (0.5 mmoles) et de diisopropyléthylamine (4 mmoles) dans le DMF est agitée à température ambiante durant 15 minutes. Le chlorhydrate de spiroisoxazoline (1 mmole) y est ensuite ajouté. La réaction étant achevée au bout de 2h de d'agitation (suivi par LC/MS), le solvant est évaporé sous pression réduite. Le résidu obtenu est repris dans le dichlorométhane et est lavé successivement à l'aide d'HCl 1N (2 fois), d'une solution saturée en NaHCO₃ et de saumure. La phase organique est ensuite séchée sur MgSO₄ et concentrée à sec. Le composé est alors purifié par HPLC préparative.

### Evaluation de l'activité des composés

### Le test cellulaire de potentialisation de l'éthionamide

Le test utilisé permet de vérifier que ces composés sont capables de potentialiser l'activité bactéricide de l'éthionamide sur des macrophages infectés par *M*. *tuberculosis.* Ce test est un test de « High Content Screening » (HCS) ou criblage à contenu dense. Les tests HCS sont réalisés sur des cultures cellulaires qui permettent d'étudier certains caractères phénotypiques d'un microorganisme (bactérie par exemple) dans un environnement donné. Les changements phénotypiques observés peuvent aller de l'augmentation (ou la diminution) de la production de certaines protéines marquées à la modification de la morphologie du microorganisme étudié. La méthode est décrite dans la publication suivante : "Ethionamide Boosters: Synthesis, Biological Activity, and Structure-Activity Relationships of a Series of 1,2,4-Oxadiazole EthR Inhibitors", M. Flipo et al., Journal of Médicinal Chemistry, 2011, 54(8), 2994-3010.

Ce test a pour but de déterminer la concentration en ligand nécessaire pour potentialiser dix fois l'activité de l'éthionamide (ETH).

Pour mesurer la concentration de ligand nécessaire pour potentialiser dix fois l'activité de l'ETH, on se place à une concentration constante en éthionamide (0.1 µg/mL ce qui correspond au 1/10^{ème} de sa CMI₉₉). En faisant varier la concentration en ligand on peut déterminer la concentration nécessaire pour inhiber 50% de la pousse bactérienne, c'est-à-dire la concentration nécessaire pour potentialiser dix fois l'activité de l'éthionamide. Cette concentration sera notée EC₅₀.

### Mesure de la solubilité

40 µL d'une solution à 10 mM dans le DMSO de l'échantillon est ajoutée à 1.96 mL de MeOH ou de PBS à pH 7.4. Les échantillons sont alors agités pendant 24h à TA, centrifugés pendant 5 min puis filtrés sur des filtres de taille 0.45 µm. 20 µL de chaque solution est alors ajoutée à 180 µL de MeOH puis analysée par LC-MS. La solubilité est déterminée comme le ratio des aires des signaux de masses PBS/MeOH.

### ACTIVITES BIOLOGIQUES MESUREES

Le tableau I suivant regroupe les formules des composés (1-53 et 55-59) de l'invention testés ainsi que les valeurs des EC₅₀ mesurées expérimentalement selon le protocole précité.

**TABLEAU I**

| **N°** | **Composé** | **RMN** | **[MH]⁺** | **EC₅₀ (µM)** |
|---|---|---|---|---|
| **4** | BDM_44636 326.32 g/mol | ¹H (CD₂Cl₂, 300 MHz): δ*(ppm)* 2.22 (m, 1H), 2.52 (m, 5H), 3.48 (m, 2H), 3.61-3.98 (m, 4H), 7.47 (m, 3H), 7.70 (m, 2H). | 327 | 0.53 |
| | 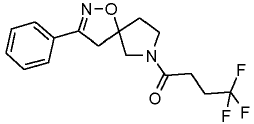 | ¹³C(CD₂Cl₂, 75MHz): δ*(ppm)* 26.6 and 27.1 (q, *J*=2.9Hz), 29.0 (q, *J*=29.5Hz), 35.6, 37.2, 41.2 and 41.6, 44.9, 45.5, 55.7, 56.5, 89.7 and 91.2, 126.5, 128.7, 129.1, 130.3, 127.6 (q, *J*=271.1Hz), 156.5 and 156.6, 167.9 and 168.2. | | |
| | | ¹⁹F (CD₂Cl₂, 282 MHz): δ*(ppm)* -67.00, -66.98. | | |
| **5** | BDM_44635 312.29 g/mol | ¹H (CD₂Cl₂, 300 MHz): δ*(ppm)* 2.26-2.56 (m, 4H), 3.65 (s, 2H), 4.21 and 4.34 (2d, 2H, *J*=11.1 and 10.5Hz), 4.32 and 4.50 (2d, 2H, *J*=9.3 and 9.3Hz), 7.46 (m, 3H), 7.68 (m, 2H). | 313 | 0.11 |
| | 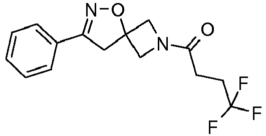 | ¹³C (CD₂Cl₂, 75 MHz): δ*(ppm)* 24.3 (q, *J*=3.1 Hz), 28.8 (q, *J*=28.4Hz), 44.5, 61.5, 63.7, 80.2, 126.6, 128.8, 130.5, 127.1 (q, *J*=279.5Hz), 156.8, 169.1. | | |
| | | ¹⁹F (CD₂Cl₂, 282 MHz): δ*(ppm)* -37.12. | | |
| **6** | BDM_41774 346.37 g/mol | ¹H (CDCl₃, 300 MHz): δ *(ppm)* 1.69 (m, 2H), 1.94 (m, 2H), 2.46 (m, 4H), 3.12 (s, 2H), 3.24 (m, 1H), 3.52 (m, 2H), 4.26 (m, 1H), 7.04 (q, 1H, *J*=5.1 and 3.6Hz), 7.16 (dd, 1H, *J*=3.6 and 1.1Hz), 7.38 (dd, 1H, *J*=5.1 and 1.1Hz). | 347 | 0.03 |
| | 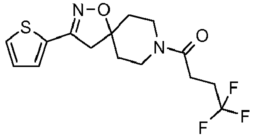 | | | |
| **7** | BDM_41776 347.36 g/mol | ¹H (CDCl₃, 300 MHz): δ *(ppm)* 1.72 (m, 2H), 1.95 (m, 2H), 2.45 (m, 4H), 3.29 (s, 2H), 3.33 (m, 1H), 3.58 (m, 2H), 4.15 (m, 1H), 7.41 (d, 1H, *J*=3.2Hz), 7.86 (d, 1H, *J*=3.2Hz). | 348 | 0.04 |
| | 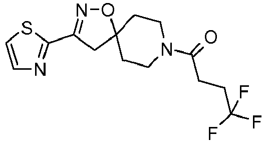 | ¹³C (CDCl₃, 75 MHz): δ (*ppm*)25.9, 29.6 (q, J=29.1 Hz), 35.4, 36.2, 37.9, 39.0, 45.2, 85.7, 121.3, 127.1 (q, J=274.0Hz), 143.5, 153.8, 157.7, 168.0. | | |
| **8** | BDM_44783 | ¹H (CD₂Cl₂, 300 MHz): δ*(ppm)* 0.91 (d, 6H, *J*=6.6 Hz), 1.40-1.58 (m, 3H), 1.71 (m, 2H), 1.91 (m, 2H), 3.12 (s, 2H), 3.35 (m, 1H), 3.58 (m, 2H), 4.04 (m, 1H), 7.07 (m, 2H), 7.17 (m, 2H), 7.40 (m, 2H). | 321 | 0.13 |
| | 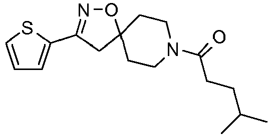 | | | |
| **9** | 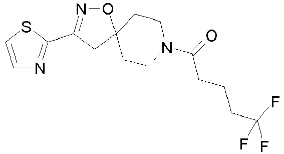 BDM_44780 | ¹H (CDCl₃, 300 MHz): δ *(ppm)* 1.79 (m, 2H), 1.90 (m, 4H), 2.16 (m, 2H), 2.42 (t, 2H, J=7.2Hz), 3.29 (s, 2H), 3.33 (m, 1H), 3.58 (m, 2H), 4.03 (m, 1H), 7.45 (d, 1H, *J*=3.2Hz), 7.86 (d, 1H, *J*=3.2Hz). | 362 | 0.07 |
| **10** | BDM_41420 340.35 g/mol | ¹H (CD₂Cl₂, 300 MHz): δ (ppm) 1.36-1.88 (m, 2H), 1.90-2.04 (m, 2H), 2.45-2.61 (m, 4H), 3.16 (s, 2H), 3.42 (m, 1H), 3.56 (m, 2H), 4.18 (m, 1H), 7.47 (m, 3H), 7.70 (m, 2H). | 341 | 0.02 |
| | 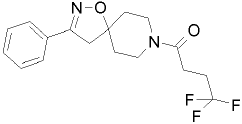 | ¹³C (CD₂Cl₂, 75 MHz): δ (ppm) 25.8, 29.5 (q, J=39.1 Hz), 35.5 and 36.2, 39.4 and 42.6, 45.3, 84.1, 126.5, 128.7 and 130.0, 127.6 (q, J=275.3 Hz), 156.4, 167.8. | | |
| | | | | |
| **11** | BDM_41781 354.38 g/mol | ¹H (CDCl₃, 300 MHz): δ*(ppm)* 1.69 (m, 2H), 1.94 (m, 2H), 2.38 (s, 3H), 2.45 (m, 4H), 3.10 (s, 2H), 3.25 (m, 1H), 3.53 (m, 2H), 4.26 (m, 1H), 7.19 (d, 2H, *J*=7.9 Hz), 7.54 (d, 2H, *J*=5.0 Hz). | 355 | 2.50 |
| | 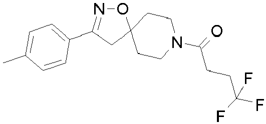 | | | |
| **12** | BDM_41789 382.43 g/mol | ¹H (CDCl₃, 300 MHz): δ*(ppm)* 1.25 (d, 6H, J=6.9Hz), 1.69 (m, 2H), 1.94 (m, 2H), 2.43 (m, 4H), 2.89 (qp, 1H, J=13.8 and 6.9Hz), 3.11 (s, 2H), 3.26 (m, 1H), 3.54 (m, 2H), 4.26 (m, 1H), 7.26 (d, 2H, J=8.2Hz), 7.56 (dt, 2H, J=8.3 and 1.9 Hz). | 383 | ND |
| | 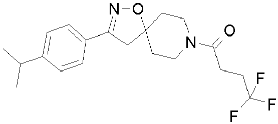 | | | |
| **13** | BDM_41793 396.46 g/mol | ¹H (CDCl₃, 300 MHz): δ*(ppm)* 1.30 (s, 9H), 1.66 (m, 2H), 1.92 (m, 2H), 2.42 (m, 4H), 3.08 (s, 2H), 3.22 (m, 1H), 3.50 (m, 2H), 4.24 (m, 1H), 7.38 (d, 2H, *J*=8.6Hz), 7.54 (d, 2H, *J*=8.6Hz). | 397 | ND |
| | 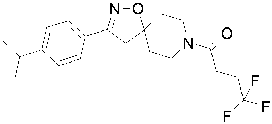 | | | |
| **14** | BDM_41938 370.37 g/mol | ¹H (CDCl₃, 300 MHz): δ*(ppm)* 1.66 (m, 2H), 1.94 (m, 2H), 2.40 (m, 4H), 3.22 (s, 2H), 3.25 (m, 1H), 3.52 (m, 2H), 3.84 (s, 3H), 4.23 (m, 1H), 6.91 (m, 2H), 7.35 (td, 1H, *J*=7.5 and 1.7Hz), 7.70 (dd, 1H, *J*=7.7 and 1.7Hz). | 371 | 0.02 |
| | 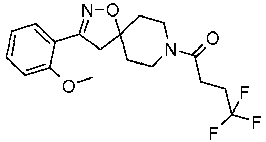 | | | |
| **15** | BDM_43097 370.37 g/mol | ¹H (CD₂Cl₂, 300 MHz): δ*(ppm)* 1.71 (m, 2H), 1.94 (m, 2H), 2.55 (m, 4H), 3.14 (s, 2H), 3.38 (m, 1H), 3.58 (m, 2H), 3.85 (s, 3H), 4.17 (m, 1H), 6.99 (m, 1H), 7.21 (m, 2H), 7.35 (t, 1H, *J*=7.8Hz). | 371 | 0.29 |
| | 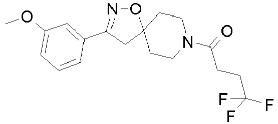 | | | |
| **16** | BDM_41935 370.37 g/mol | ¹H (CDCl₃, 300 MHz): δ*(ppm)* 1.68 (m, 2H), 1.94 (m, 2H), 2.46 (m, 4H), 3.09 (s, 2H), 3.25 (m, 1H), 3.53 (m, 2H), 3.83 (s, 3H), 4.26 (m, 1H), 6.90 (m, 1H), 7.56 (m, 1H). | 371 | ND |
| | 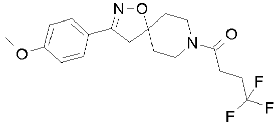 | | | |
| **17** | BDM_43116 400.40 g/mol | ¹H (CD₂Cl₂, 300 MHz): δ*(ppm)* 1.82 (m, 2H), 1.95 (m, 2H), 2.55 (m, 4H), 3.13 (s, 2H), 3.43 (m, 1H), 3.65 (m, 2H), 3.89 (s, 6H), 4.15 (m, 1H), 6.90 (d, 1H, *J*=8.4Hz), 7.07 (dd, 1H, *J*=8.3 and 2.0Hz), 7.35 (d, 1 H, *J*=1.9Hz). | 401 | ND |
| | 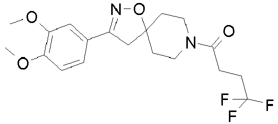 | | | |
| **18** | BDM_41797 384.36 g/mol | ¹H (CDCl₃, 300 MHz): δ*(ppm)* 1.69 (m, 2H), 1.95 (m, 2H), 2.48 (m, 4H), 3.08 (s, 2H), 3.25 (m, 1H), 3.54 (m, 2H), 4.27 (m, 1H), 6.02 (s, 2H), 6.81 (d, 1H, *J*=8.1Hz), 6.99 (dd, 1H, *J*=8.1 and 1.7Hz), 7.27 (d, 1 H, *J*=1.5Hz). | 385 | 1.30 |
| | 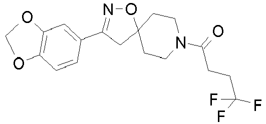 | | | |
| **19** | BDM_41791 424.35 g/mol | ¹H (CDCl₃, 300 MHz): δ*(ppm)* 1.70 (m, 2H), 1.94 (m, 2H), 2.45 (m, 4H), 3.10 (s, 2H), 3.24 (m, 1H), 3.53 (m, 2H), 4.26 (m, 1H), 7.24 (m, 2H), 7.66 (dt, 2H, *J*=9.0 and 2.7Hz). | 425 | <20 |
| | 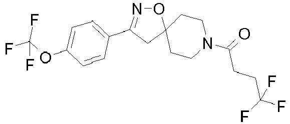 | | | |
| **20** | BDM_41931 374.79 g/mol | ¹H (CDCl₃, 300 MHz): δ*(ppm)* 1.71 (m, 2H), 2.01 (m, 2H), 2.42 (m, 4H), 3.22 (s, 2H), 3.28 (m, 1H), 3.55 (m, 2H), 4.27 (m, 1H), 7.28 (m, 3H), 7.61 (dd, 1H, *J*=7.3 and 1.9Hz). | 375 | 0.03 |
| | 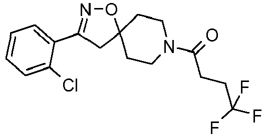 | | | |
| **21** | BDM_41779 374.79 g/mol | ¹H (CDCl₃, 300 MHz): δ*(ppm)* 1.69 (m, 2H), 1.95 (m, 2H), 2.46 (m, 4H), 3.09 (s, 2H), 3.24 (m, 1H), 3.53 (m, 2H), 4.26 (m, 1H), 7.35 (d, 2H, *J*=13.2Hz), 7.53 (d, 2H, *J*=13.5Hz). | 375 | 5.80 |
| | 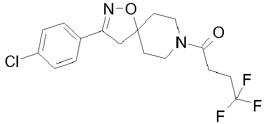 | | | |
| **22** | BDM_41783 358.34 g/mol | ¹H (CDCl₃, 300 MHz): δ*(ppm)* 1.69 (m, 2H), 1.95 (m, 2H), 2.43 (m, 4H), 3.10 (s, 2H), 3.24 (m, 1H), 3.53 (m, 2H), 4.27 (m, 1H), 7.05 (m, 2H), 7.60 (m, 2H). | 359 | 0.50 |
| | 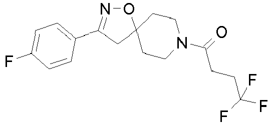 | | | |
| **23** | BDM_41775 408.35 g/mol | ¹H (CDCl₃, 300 MHz): δ*(ppm)* 1.72 (m, 2H), 1.97 (m, 2H), 2.47 (m, 4H), 3.15 (s, 2H), 3.27 (m, 1H), 3.55 (m, 2H), 4.28 (m, 1H), 7.52 (t, 1H, *J*=7.7Hz), 7.66 (d, 1H, *J*=7.8Hz), 7.85 (m, 1H). | 409 | 0.40 |
| | 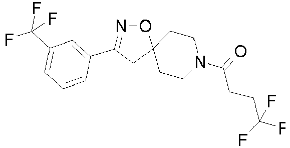 | | | |
| **24** | BDM_41933 408.35 g/mol | ¹H (CDCl₃, 300 MHz): δ*(ppm)* 1.72 (m, 2H), 1.98 (m, 2H), 2.47 (m, 4H), 3.14 (s, 2H), 3.27 (m, 1H), 3.55 (m, 2H), 4.29 (m, 1H), 7.66 (d, 1H, *J*=8.3Hz), 7.75 (d, 1H, *J*=8.2Hz). | 409 | <20 |
| | 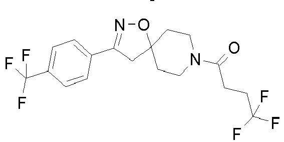 | | | |
| **25** | BDM_43117 418.44 g/mol | ¹H (CD₂Cl₂, 300 MHz): δ*(ppm)* 1.74 (m, 2H), 1.95 (m, 2H), 2.45 (m, 4H), 3.07 (s, 3H), 3.17 (s, 2H), 3.34 (m, 1H), 3.54 (m, 2H), 4.15 (m, 1H), 7.84 (ddt, 2H, *J*=8.7 and 2.0Hz), 7.96 (ddt, 2H, *J*=8.6 and 1.8Hz). | 419 | ND |
| | 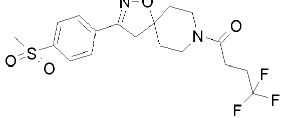 | | | |
| **26** | BDM_41785 390.41 g/mol | ¹H (CDCl₃, 300 MHz): δ*(ppm)* 1.73 (m, 2H), 1.99 (m, 2H), 2.47 (m, 4H), 3.24 (s, 2H), 3.28 (m, 1H), 3.55 (m, 2H), 4.28 (m, 1H), 7.48 (m, 2H), 7.81 (m, 4H), 7.94 (m, 1H). | 391 | ND |
| | 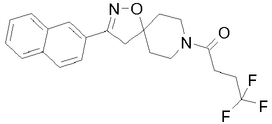 | | | |
| **27** | BDM_41795 346.40 g/mol | ¹H (CDCl₃, 300 MHz): δ*(ppm)* 1.16 (m, 5H), 1.54 (m, 3H), 1.76 (m, 6H), 2.34 (m, 5H), 2.66 (s, 2H), 3.16 (m, 1H), 3.45 (m, 2H), 4.19 (m, 1 H). | 347 | 0.08 |
| | 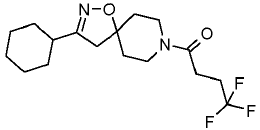 | | | |
| **28** | BDM_41787 332.37 g/mol | ¹H (CDCl₃, 300 MHz): δ*(ppm)* 1.54 (m, 8H), 1.72 (m, 4H), 2.44 (m, 4H), 2.68 (s, 2H), 2.85 (m, 1H), 3.17 (m, 1H), 3.45 (m, 2H), 4.20 (m, 1 H). | 333 | 0.08 |
| | 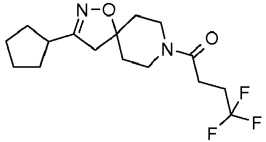 | | | |
| **29** | BDM_43100 354.38 g/mol | ¹H (CD₂Cl₂, 300 MHz): δ*(ppm)* 1.54 (m, 2H), 1.77 (m, 2H), 2.44 (m, 4H), 3.25 (m, 1H), 3.54 (m, 2H), 3.68 (s, 2H), 4.02 (m, 1H), 7.24 (m, 5H). | 355 | 0.02 |
| | 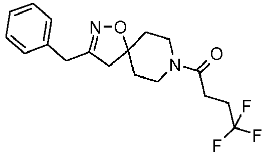 | | | |
| **30** | BDM_41777 330.31 g/mol | ¹H (CDCl₃, 300 MHz): δ*(ppm)* 1.68 (m, 2H), 1.93 (m, 2H), 2.44 (m, 4H), 3.08 (s, 2H), 3.25 (m, 1H), 3.53 (m, 2H), 4.24 (m, 1H), 6.84 (m, 1H), 6.70 (dd, 1H, *J*=3.4 and 0.6Hz), 7.51 (m, 1H). | 331 | 0.05 |
| | 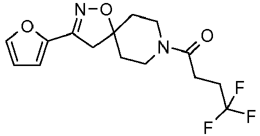 | | | |
| **31** | BDM_41778 341.34 g/mol | ¹H (CDCl₃, 300 MHz): δ*(ppm)* 1.72 (m, 2H), 1.94 (m, 2H), 2.40 (m, 4H), 3.29 (s, 2H), 3.38 (m, 1H), 3.48 (m, 2H), 4.11 (m, 1H), 7.28 (m, 1H), 7.70 (td, 1H, *J*=7.6 and 1.8Hz), 8.00 (dt, 1H, *J*=8.0 and 1.1 Hz), 8.58 (m, 1H). | 342 | 0.15 |
| | 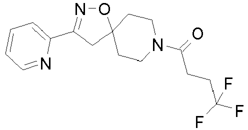 | | | |
| **32** | BDM_41418 | ¹H (CD₂Cl₂, 300 MHz): δ*(ppm)* 0.82 (d, 6H, *J*=6.3 Hz), 1.36-1.45 (m, 2H), 1.56 (m, 1H), 1.71 (m, 2H), 1.87 (m, 2H), 3.02 (s, 2H), 3.27 (m, 1H), 3.42 (m, 2H), 3.95 (m, 1H), 7.33 (m, 3H), 7.55 (m, 2H). | 315 | 5.00 |
| | 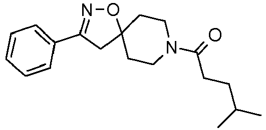 | | | |
| **33** | BDM_41796 | ¹H (CDCl₃): δ *(ppm)* 0.90 (d, 6H, J = 6.28Hz), 1.24 (m, 3H), 1.43 (m, 5H), 1.70 (m, 5H), 1.91 (m, 2H), 2.02 (m, 2H), 2.31 (m, 2H), 2.71 (m, 1H), 2.81 (m, 1H), 3.10 (m, 2H), 3.87 (m, 1H), 4.47 (m, 1H). | 334 | ND |
| | 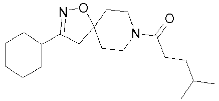 | | | |
| **34** | BDM_41788 | ¹H (CDCl₃): δ *(ppm)* 0.90 (d, 6H, J = 6.29Hz), 1.47 (m,3H), 1.63 (m, 8H), 1.91 (m, 4H), 2.30 (m, 2H), 2.83 (m, 1H), 3.10 (m, 3H), 3.87 (m, 1 H), 4.48 (m, 1H). | 320 | ND |
| | 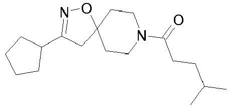 | | | |
| **35** | BDM_43118 | ¹H (CD₂Cl₂): δ *(ppm)* 0.93 (d, 6H,J = 6.40Hz), 1.48 (m, 3H), 1.66 (m, 2H), 1.84 (m, 2H), 2.33 (m, 2H), 3.07 (s, 3H), 3.16 (s, 2H), 3.35 (m, 1H), 3.55 (m, 2H), 4.08 (m, 1H), 7.84 (ddt, 2H, J = 8.64Hz, J = 1.98Hz), 7.96 (ddt, 2H, J = 8.63Hz, J = 1.81Hz). | 393 | ND |
| | 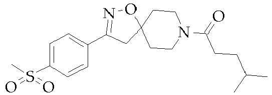 | | | |
| **36** | BDM_41780 | ¹H (CDCl₃): δ *(ppm)* 0.89 (d, 6H, J = 6.26Hz), 1.47 (m, 3H), 1.62 (m, 2H), 1.91 (m, 2H), 2.30 (m, 2H), 3.06 (s, 2H), 3.25 (m, 1H), 3.50 (m, 2H), 4.19 (m, 1H), 7.33 (m, 2H), 7.53 (m, 2H). | 349 | ND |
| | 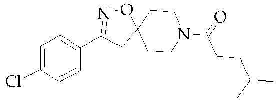 | | | |
| **37** | BDM_41782 | ¹H (CDCl₃): δ *(ppm)* 0.93 (d, 6H, J = 6.28Hz), 1.51 (m, 3H), 1.25 (m, 2H), 1.94 (m, 2H), 2.31 (m, 2H), 2.39 (s, 3H), 3.11 (s, 2H), 3.29 (m, 1H), 3.54 (m, 2H), 4.21 (m, 1H), 7.21 (d, 2H, J = 7.97Hz), 7.54 (d, 2H, J = 8.14Hz). | 329 | ND |
| | 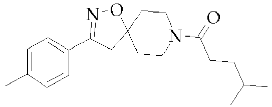 | | | |
| **38** | BDM_41784 | ¹H (CDCl₃): δ *(ppm)* 0.90 (d, 6H, J = 6.28Hz), 1.48 (m, 3H), 1.64 (m, 2H), 1.92 (m, 2H), 2.32 (m, 2H), 3.08 (s, 2H), 3.26 (m, 1H), 3.52 (m, 2H), 4.20 (m, 1H), 7.06 (m, 2H), 7.60 (m, 2H). | 333 | ND |
| | 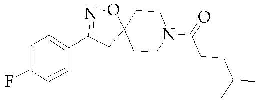 | | | |
| **39** | BDM_41786 | ¹H (CDCl₃): δ *(ppm)* 0.91 (d, 6H, J = 6.27Hz), 1.49 (m, 3H), 1.64 (m, 2H), 1.95 (m, 2H), 2.33 (m, 2H), 3.22 (s, 2H), 3.30 (m, 1H), 3.54 (m, 2H), 4.20 (m, 1H), 7.47 (m, 2H), 7.82 (m, 4H), 7.94 (m, 1H). | 365 | ND |
| | 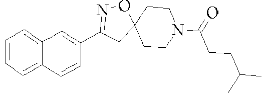 | | | |
| **40** | BDM_41790 | ¹H (CDCl₃): δ *(ppm)* 0.93 (d, 6H, J = 6.28Hz), 1.26 (d, 6H, J = 6.91Hz), 1.49 (m, 3H), 1.66 (m, 2H), 1.94 (m, 2H), 2.34 (m, 2H), 2.90 (qp, 1H, J = 13.79Hz, J = 6.90Hz), 3.11 (s, 2H), 3.29 (m, 1H), 3.54 (m, 2H), 4.23 (m, 1H), 7.27 (d, 2H, J = 8.21Hz), 7.58 (d, 2H, J = 8.32Hz). | 357 | ND |
| | 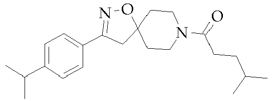 | | | |
| **41** | BDM_41792 | ¹H (CDCl₃): δ *(ppm)* 0.93 (d, 6H, J = 6.28Hz), 1.51 (m, 3H), 1.66 (m, 2H), 1.95 (m, 2H), 2.34 (m, 2H), 3.11 (s, 2H), 3.28 (m, 1H), 3.54 (m, 2H), 4.25 (m, 1H), 7.28 (d, 2H, J = 7.98Hz), 7.67 (dt, 2H, J = 6.85Hz, J = 2.02Hz). | 399 | ND |
| | 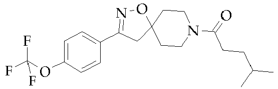 | | | |
| **42** | BDM_41794 | ¹H (CDCl₃): δ *(ppm)* 0.91 (d, 6H, J = 6.27Hz), 1.32 (s, 9H), 1.48 (m, 3H), 1.62 (m, 2H), 1.92 (m, 2H), 2.32 (m, 2H), 3.09 (s, 2H), 3.26 (m, 1H), 3.53 (m, 2H), 4.21 (m, 1 H), 7.41 (d, 2H, J = 8.48Hz), 7.57 (d, 2H, J = 8.40Hz). | 371 | ND |
| | 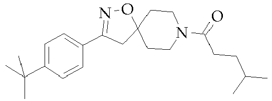 | | | |
| **43** | BDM_41796 | ¹H (CDCl₃): δ *(ppm)* 0.88 (d, 6H, J = 6.30Hz), 1.23 (m, 5H), 1.45 (m, 6H), 1.69 (m, 6H), 2.28 (m, 3H), 2.64 (s, 2H), 3.17 (m, 1H), 3.43 (m, 2H), 4.13 (m, 1H). | 321 | ND |
| | 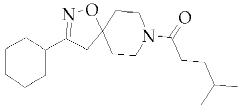 | | | |
| **44** | BDM_41798 | ¹H (CDCl₃): δ *(ppm)* 0.93 (d, 6H, J = 6.30Hz), 1.49 (m, 2H), 1.66 (m, 2H), 1.78 (m, 2H), 1.94 (m, 2H), 2.34 (m, 2H), 3.07 (s, 2H), 3.27 (m, 1H), 3.54 (m, 2H), 4.22 (m, 1H), 6.02 (s, 2H), 6.81 (d, 1H, J = 8.30Hz), 6.99 (dd, 1 H, J = 8.06Hz, J = 1.70Hz), 7.28 (d, 1H, J = 1.64Hz). | 359 | ND |
| | 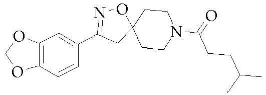 | | | |
| **45** | BDM_41799 | ¹H (CDCl₃): δ *(ppm)* 0.91 (d, 6H, J = 6.29Hz), 1.48 (m, 3H), 1.64 (m, 2H), 1.93 (m, 2H), 2.33 (m, 2H), 3.28 (s, 2H), 3.42 (m, 1H), 3.59 (m, 2H), 4.04 (m, 1H), 7.27 (m, 1H), 7.70 (td, 1H, J = 7.67Hz, J = 1.77Hz), 8.00 (dt, 1H, J = 7.99Hz, J = 1.03Hz), 8.58 (m, 1H). | 316 | 1.30 |
| | 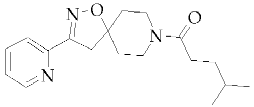 | | | |
| **46** | BDM_41932 | ¹H (CDCl₃): δ *(ppm)* 0.92 (d, 6H, J = 6.29Hz), 1.49 (m, 3H), 1.65 (m, 2H), 1.98 (m, 2H), 2.33 (m, 2H), 3.27 (s, 2H), 3.30 (m, 1H), 3.54 (m, 2H), 4.19 (m, 1H), 7.27 (m, 3H), 7.61 (dd, 1H, J = 7.29Hz, J = 1.81Hz). | 349 | 1.30 |
| | 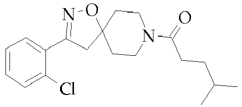 | | | |
| **47** | BDM_41934 | ¹H (CDCl₃): δ *(ppm)* 0.90 (d, 6H, J = 6.26Hz), 1.48 (m, 3H), 1.70 (m, 2H), 1.93 (m, 2H), 2.31 (m, 2H), 3.10 (s, 2H), 3.26 (m, 1H), 3.49 (m, 2H), 4.21 (m, 1H), 7.63 (d, 1H, J = 8.29Hz), 7.73 (d, 1H, J = 8.24Hz). | 383 | ND |
| | 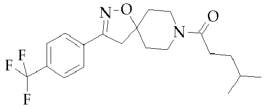 | | | |
| **48** | BDM_41936 | ¹H (CDCl₃): δ *(ppm)* 0.90 (d, 6H, J = 6.26Hz), 1.48 (m, 3H), 1.61 (m, 2H), 1.91 (m, 2H), 2.31 (m, 2H), 3.07 (s, 2H), 3.26 (m, 1H), 3.51 (m, 2H), 3.82 (s, 3H), 4.19 (m, 1H), 6.89 (m, 1H), 7.55 (m, 1H). | 345 | ND |
| | 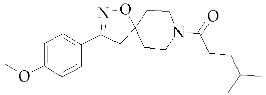 | | | |
| **49** | BDM_41939 | ¹H (CDCl₃): δ *(ppm)* 0.91 (d, 6H, J = 6.30Hz), 1.48 (m, 3H), 1.62 (m, 2H), 1.92 (m, 2H), 2.32 (m, 2H), 3.22 (s, 2H), 3.27 (m, 1H), 3.57 (m, 2H), 3.85 (s, 3H), 4.18 (m, 1H), 6.92 (m, 2H), 7.35 (td, 1H, J = 7.46Hz, J = 1.74Hz), 7.70 (dd, 1H, J = 7.68Hz, J = 1.72Hz). | 345 | 0.63 |
| | 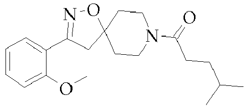 | | | |
| **50** | BDM_43103 | ¹H (CD₂Cl₂): δ *(ppm)* 1.72 (m, 2H), 1.86 (m, 4H), 2.15 (m, 2H), 2.43 (m, 2H), 3.29 (s, 2H), 3.34 (m, 1H), 3.52 (m, 2H), 4.10 (m, 1H), 7.33 (m, 2H), 7.46 (m, 1H), 7.61 (m, 1H). | 389 | 0.77 |
| | 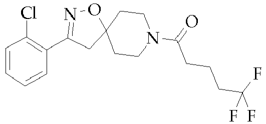 | | | |
| **51** | BDM_41941 | ¹H (MeOD): δ *(ppm)* 1.74 (m, 6H), 2.13 (m, 2H), 2.52 (t, 2H, J = 7.20Hz), 3.26 (s, 2H), 3.40 (m, 1H), 3.49 (m, 1H), 3.65 (m, 1H), 3.99 (m, 1H), 7.40 (m, 3H), 7.65 (m, 2H). | 355 | 1.00 |
| | 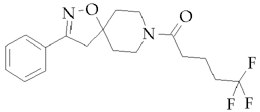 | | | |
| **52** | BDM_43119 | ¹H (CD₂Cl₂): δ *(ppm)* 1.75 (m, 2H), 1.86 (m, 4H), 2.15 (m, 2H), 2.42 (m, 2H), 3.07 (s, 3H), 3.16 (s, 2H), 3.34 (m, 1H), 3.52 (m, 2H), 4.11 (m, 1 H), 7.85 (ddt, 2H, J = 8.65Hz, J = 1.94Hz), 7.97 (ddt, 2H, J = 8.65Hz, J = 1.72Hz). | 433 | <20 |
| | 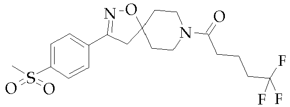 | | | |
| **53** | BDM_43092 | ¹H (CD₂Cl₂): δ *(ppm)* 1.72 (m, 2H), 1.94 (m, 2H), 3.14 (s, 2H), 3.35 (m, 1H), 3.59 (m, 1H), 3.67 (m, 1H), 4.13 (m, 1H), 4.73 (d, 1H, J = 3.80Hz), 6.95 (m, 3H), 7.30 (m, 2H), 7.42 (m, 3H), 7.46 (m, 2H). | 351 | <20 |
| | 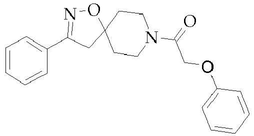 | | | |
| **54** | BDM_43113 | ¹H (CD₂Cl₂): δ *(ppm)* 1.76 (m, 1H), 1.85 (m, 3H), 3.13 (s, 3H), 3.16 (s, 2H), 3.31 (m, 1H), 3.62 (m, 1H), 3.76 (m, 1H), 4.05 (m, 2H), 4.27 (m, 1H), 7.42 (m, 3H), 7.64 (m, 2H). | 337 | <20 |
| | 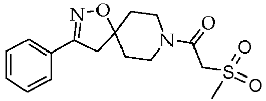 | | | |
| **55** | BDM_43114 | ¹H (CD₂Cl₂): δ *(ppm)* 1.49 (m, 1H), 1.69 (m, 2H), 1.89 (m, 1H), 3.03 (d, 2H, J = 3.90Hz), 3.36 (m, 1H), 3.52 (m, 2H), 4.10 (m, 1H), 7.25 (m, 3H), 7.33 (m, 2H), 7.41 (m, 3H), 7.63 (m, 2H). | 335 | <20 |
| | 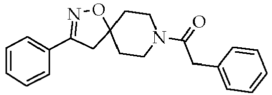 | | | |
| **56** | BDM_43095 | ¹H (CD₂Cl₂): δ *(ppm)* 1.09 (m, 3H), 1.52 (m, 10H), 2.18 (m, 1H), 2.37 (m, 2H), 3.14 (s, 2H), 3.35 (m, 1H), 3.54 (m, 2H), 4.04 (m, 1H), 7.41 (m, 3H), 7.64 (m, 2H). | 327 | <20 |
| | 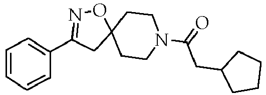 | | | |
| **57** | BDM_43102 | ¹H (CD₂Cl₂): δ *(ppm)* 1.42 (m, 1H), 1.72 (m, 2H), 1.91 (m, 2H), 2.11 (m, 1H), 2.23 (m, 5H), 3.15 (s, 2H), 3.37 (m, 1 H), 3.54 (m, 2H), 4.09 (m, 1 H), 5.72 (m, 1H), 5.78 (m, 1 H), 7.42 (m, 3H), 7.65 (m, 2H). | 325 | <20 |
| | 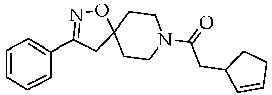 | | | |
| **58** | BDM_43259 | ¹H (CD₂Cl₂): δ *(ppm)* 1.59 (m, 2H), 1.87 (m, 2H), 3.11 (s, 2H), 3.35 (m, 1H), 3.57 (m, 2H), 3.94 (d, 2H, J = 2.40Hz), 4.13 (m, 1H), 6.92 (m, 1H), 6.97 (dd, 1H, J = 5.12Hz, J = 3.47Hz), 7.24 (dd, 1H, J = 5.20Hz, J = 1.14Hz), 7.41 (m, 3H), 7.64 (m, 2H). | 341 | <20 |
| | 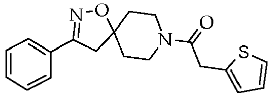 | | | |
| **59** | BDM_43104 | ¹H (CD₂Cl₂): δ *(ppm)* 1.54 (m, 1H), 1.69 (m, 1H), 2.65 (m, 2H), 2.92 (m, 2H), 3.11 (s, 2H), 3.33 (m, 1H), 3.48 (m, 2H), 4.10 (m, 1H), 7.20 (m, 5H), 7.35 (m, 3H), 7.64 (m, 2H). | 349 | 1.00 |
| | 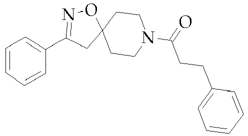 | | | |

A titre d'exemple de solubilité mesurée, le composé **10** (BDM41420) présente une solubilité égale à 150 µM mesurée selon le protocole précité.

## Revendications

1. Composés de type spiroisoxazoline de formule générale (I) : 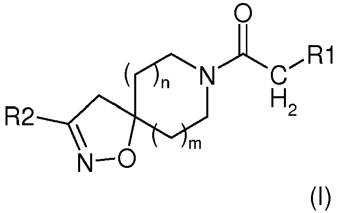 dans laquelle :
m = 0 ou 1 ;
n = 0 ou 1 ;
R1 représente un groupement choisi parmi:
les chaînes alkyles en C1-C5, linéaires ou ramifiées ;
les chaînes alkyles en C1-C5 linéaires ou ramifiées et substituées par au moins un F ;
les chaînes alkyles en C1-C3 linéaires ou ramifiées et substituées par au moins un groupement cyclique saturé ou insaturé en C3-C6 ;
les groupements cycliques saturés ou insaturés en C3-C6 ;
CN, CH₂CN, CH₂N₃ ;
Phényle, phényle substitué par Cl ou F, benzyle, benzyle substitué par Cl ou F, -O-phényle, et thiophènyle ;
R2 est choisi parmi :
phényle ;
benzyle éventuellement substitué par Cl ou F ;
naphthalènyle ;
phényle substitué par au moins une chaîne alkyle en C1-C4, linéaire ou ramifiée ; phényle substitué par au moins une chaîne alkyle en C1-C4 linéaire ou ramifiée et substituée par au moins un F ;
phényle substitué par au moins un groupement choisi parmi OCH₃, OCF₃, Cl, F, CH₃SO₂ et CF₃ ;
phényle dont deux atomes de carbone consécutifs sont substitués par un même groupement O-CH₂-O ;
cyclopropyle, cyclobutyle, cyclopentyle, cyclohexyle ; et
les hétérocyles aromatiques à 5 ou 6 sommets comportant au moins un atome dans le cycle choisi parmi S, N et O.

2. Composés selon la revendication 1, **caractérisés en ce que** m = n = 1.

3. Composés selon la revendication 1 ou 2, **caractérisés en ce que** R1 est choisi parmi les groupements suivants : -CH₂CF₃, -CF₂CF₃, -(CH₂)₂CF₃, -CH₂-isopropyle, cyclopropyle, cyclobutyle, -cyclopentyle, cyclopentènyle, -CH₂-cyclopropyle, -CH₂-cyclobutyle, -CH₂-cyclopentyle, phényle substitué par Cl ou F, benzyle, benzyle substitué par Cl ou F, -O-phényle, et thiophènyle.

4. Composés selon l'une quelconque des revendications 1 à 3, **caractérisés en ce que** R1 est choisi parmi -CH₂CF₃ et -(CH₂)₂CF₃.

5. Composés selon l'une quelconque des revendications précédentes, **caractérisés en ce que** R2 est phényle ou phényle substitués en ortho par OCH₃, OCF₃, Cl, F ou CF₃.

6. Composés selon l'une quelconque des revendications 1 à 4, **caractérisés en ce que** R2 est choisi parmi les hétérocycles aromatiques à 5 ou 6 sommets qui comportent au moins un atome choisi parmi les atomes S, N et O adjacent à l'atome de l'hétérocycle lié au carbone en alpha de l'azote du cycle 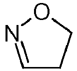 de la formule (I).

7. Composés selon la revendication 6, **caractérisés en ce que** R2 est choisi parmi les groupements suivants :

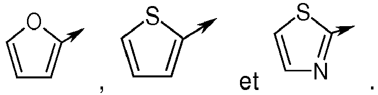

8. Composés selon l'une quelconque des revendications 1 à 4, **caractérisés en ce que** R2 est choisi parmi les groupements suivants :

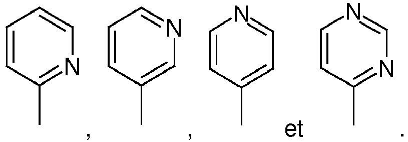

9. Composés selon la revendication 1 **caractérisés en ce qu'**ils sont choisis parmi les composés suivants :

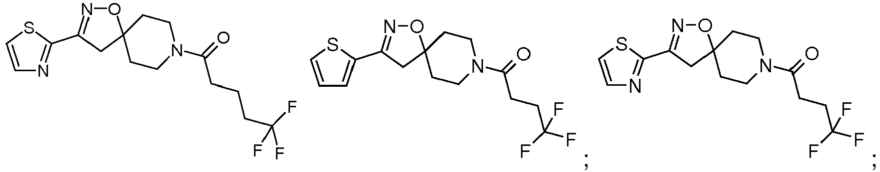

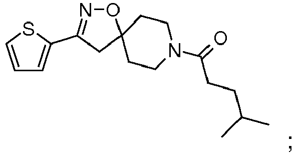

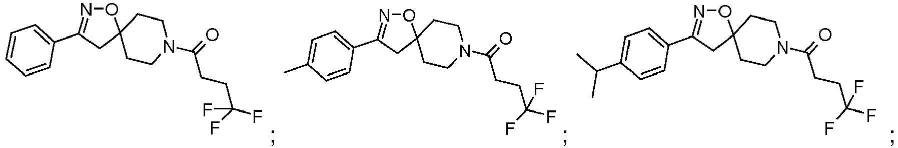

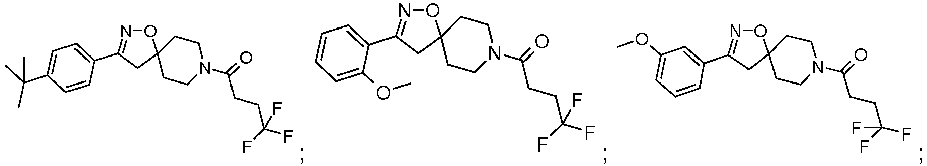

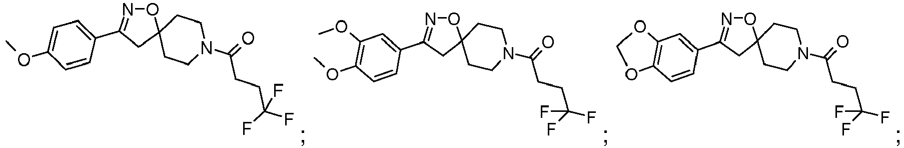

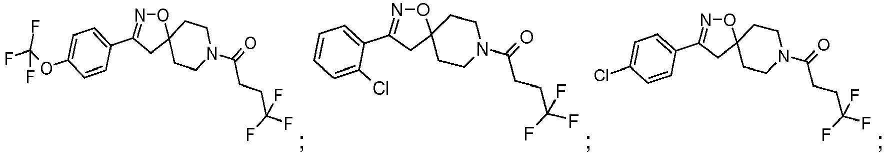

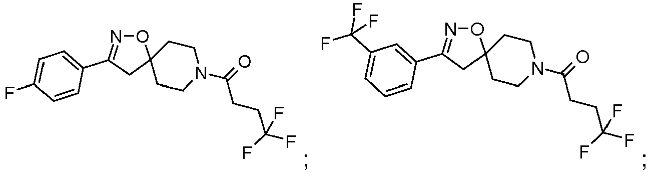

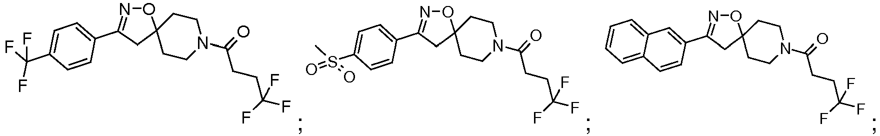

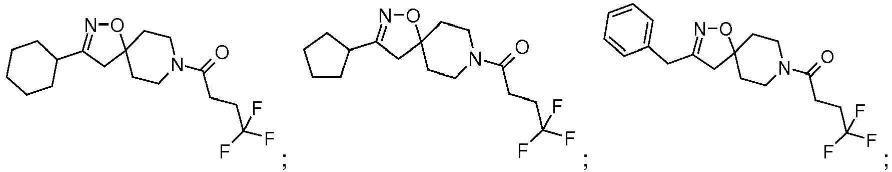

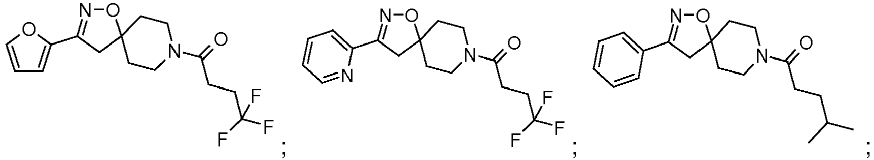

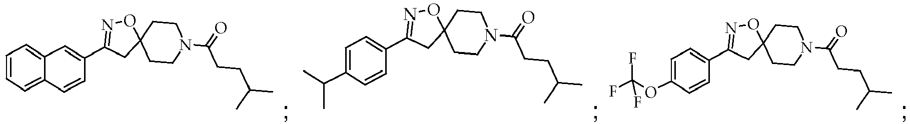

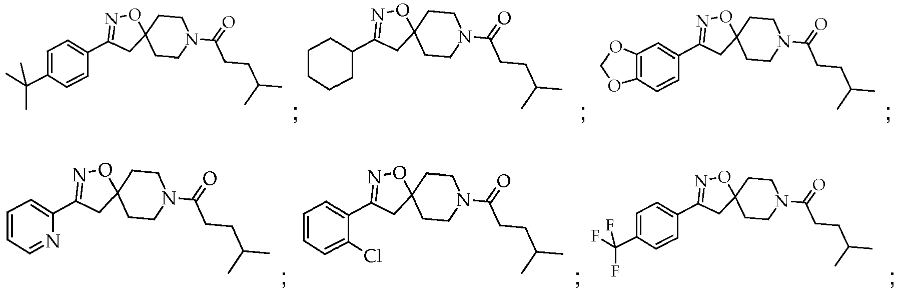

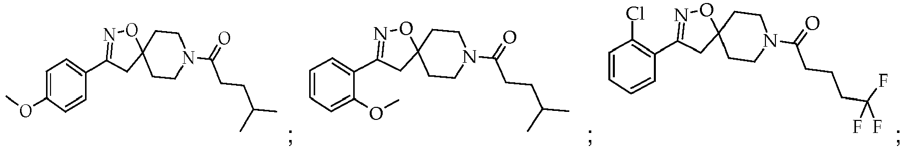

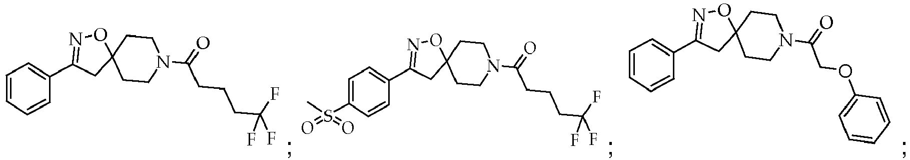

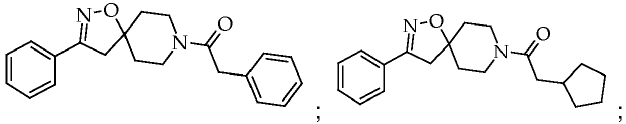

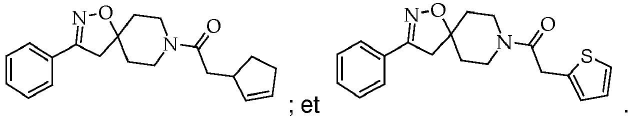

10. Composés selon l'une quelconque des revendications 1 à 9 pour leur utilisation en tant que médicament.

11. Composés selon l'une quelconque des revendications 1 à 9 pour leur utilisation dans le traitement des infections bactériennes et mycobactériennes.

12. Composés selon l'une quelconque des revendications 1 à 9 pour leur utilisation dans le traitement de la tuberculose, de la lèpre ou des mycobactérioses atypiques.

13. Composition pharmaceutique comprenant, en tant que principe actif, au moins un composé selon l'une quelconque des revendications 1 à 9 et un excipient pharmaceuticalement acceptable.

14. Composition selon la revendication 13, **caractérisée en ce qu'**elle comprend en outre, en tant que principe actif, au moins un antibiotique actif choisi parmi les antibiotiques activables par la voie enzymatique de l'EthA.

15. Composition selon la revendication 13, **caractérisée en ce qu'**elle comprend en outre, en tant que principe actif, au moins un antibiotique actif choisi parmi l'éthionamide, le prothionamide, l'isoxyl et le thiacétazone.

## Patentansprüche

1. Spiroisoxazolinverbindungen der allgemeinen Formel (I): 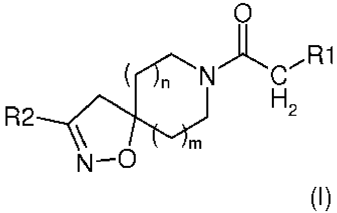 wobei:
m = 0 oder 1;
n = 0 oder 1;
R1 eine Gruppe repräsentiert, ausgewählt aus:
linearen oder verzweigten C1-C5-Alkylketten;
lineraren oder verzweigten C1-C5-Alkylketten, die mit mindestens einem F substituiert sind;
linearen oder verzweigten C1-C3-Alkylketten, die mit mindestens einer gesättigten oder ungesättigten cyclischen C3-C6-Gruppe substituiert sind;
gesättigten oder ungesättigten cyclischen C3-C6-Gruppen;
CN, CH₂CN, CH₂N₃;
Phenyl, Cl- oder F-substituiertem Phenyl, Benzyl, Cl- oder F-substituiertem Benzyl, -O-Phenyl und Thiophenyl;
R2 ausgewählt ist aus:
Phenyl;
Benzyl, gegebenenfalls substituiert mit Cl oder F;
Naphthalinyl;
Phenyl, substituiert mit mindestens einer linearen oder verzweigten C1-C4-Alkylkette;
Phenyl, substituiert mit mindestens einer linearen oder oder verzweigten C1-C4-Alkylkette und substituiert mit mindestens einem F;
Phenyl, substituiert mit mindestens einer Gruppe, ausgewählt aus OCH₃, OCF₃, Cl, F, CH₃SO₂ und CF₃;
Phenyl, in dem zwei aufeinanderfolgende Kohlenstoffatome mit der gleichen O-CH₂-O-Gruppe substituiert sind;
Cyclopropyl, Cyclobutyl, Cyclopentyl, Cyclohexyl; und
5- oder 6-gliedrigen aromatischen Heterocyclen mit mindestens einem Atom im Ring, ausgewählt aus S, N und O.

2. Verbindungen nach Anspruch 1, **dadurch gekennzeichnet, dass** m = n = 1.

3. Verbindungen nach Anspruch 1 oder 2, **dadurch gekennzeichnet, dass** R1 ausgewählt ist aus den folgenden Gruppen:
-CH₂CF₃, -CF₂CF₃, -(CH₂)₂CF₃, -CH2-lsopropyl, Cyclopropyl, Cyclobutyl, - Cyclopentyl, -Cyclopentenyl, -CH2-Cyclopropyl, -CH2-Cyclobutyl, -CH2-Cyclopentyl, mit Cl oder F substituiertes Phenyl, Benzyl, mit Cl oder F substituiertes Benzyl, -O-Phenyl und Thiophenyl.

4. Verbindungen nach einem der Ansprüche 1 bis 3, **dadurch gekennzeichnet, daß** R1 ausgewählt ist aus -CH₂CF₃ und -(CH₂)₂CF₃.

5. Verbindungen nach einem der vorhergehenden Ansprüche, **dadurch gekennzeichnet, dass** R2 Phenyl oder mit OCH₃, OCF₃, Cl, F oder CF₃ ortho-substituiertes Phenyl ist.

6. Verbindungen nach einem der Ansprüche 1 bis 4, **dadurch gekennzeichnet, daß** R2 ausgewählt ist aus 5- oder 6-gliedrigen aromatischen Heterocyclen, die mindestens ein Atom, ausgewählt aus S-, N- und O-Atomen, angrenzend an das heterocyclische Ringatom aufweisen, das an den Ringkohlenstoff Alpha zum Ringstickstoff 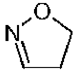 der Formel (I) gebunden ist.

7. Verbindungen nach Anspruch 6, **dadurch gekennzeichnet, dass** R2 ausgewählt ist aus den folgenden Gruppen:

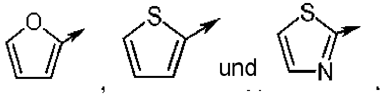

8. Verbindungen nach einem der Ansprüche 1 bis 4, **dadurch gekennzeichnet, daß** R2 ausgewählt ist aus den folgenden Gruppen:

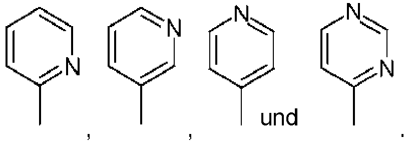

9. Verbindungen nach Anspruch 1, **dadurch gekennzeichnet, dass** sie ausgewählt sind aus den folgenden Verbindungen:

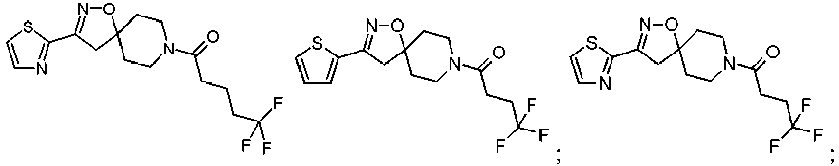

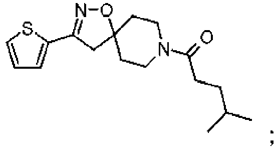

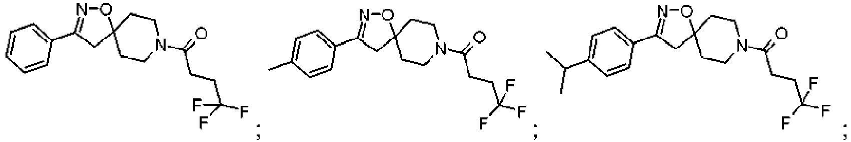

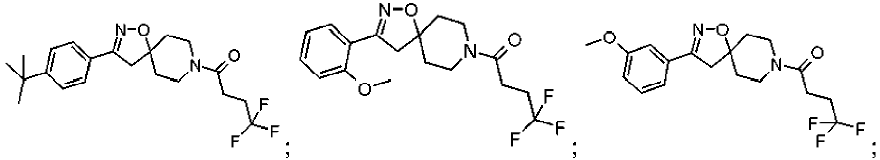

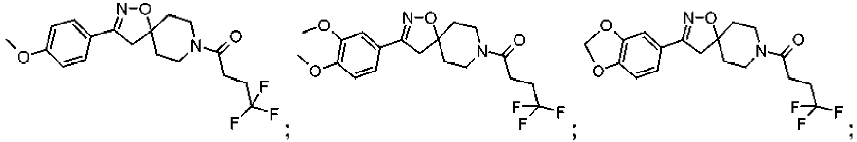

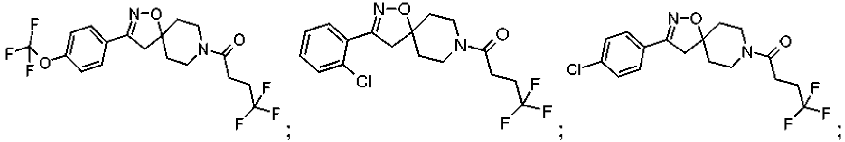

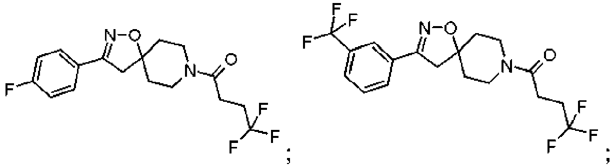

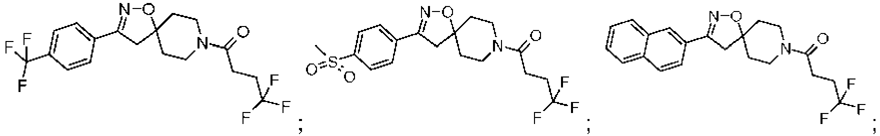

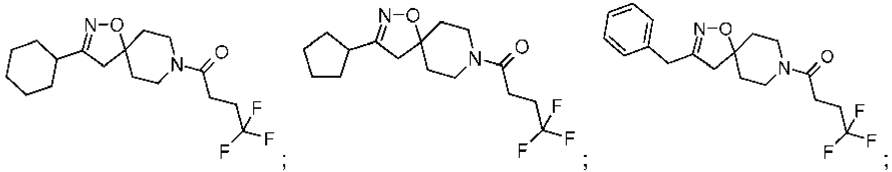

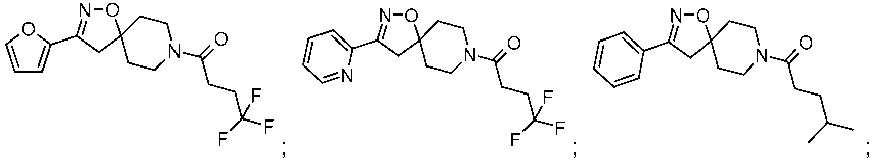

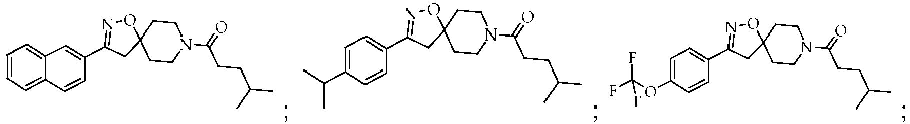

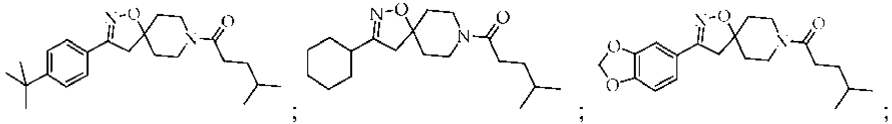

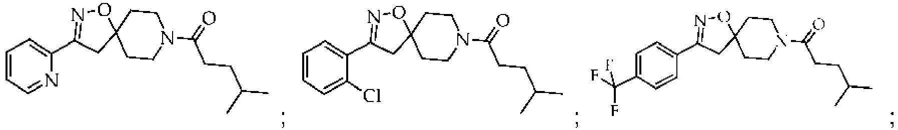

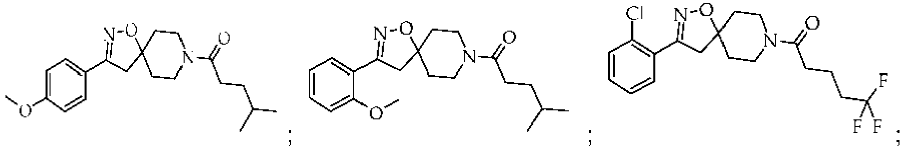

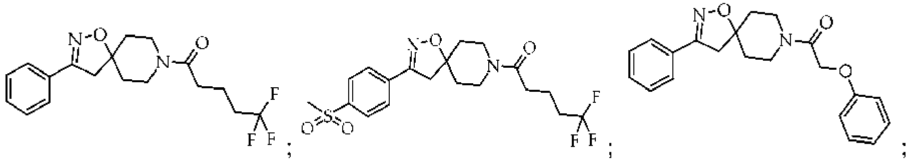

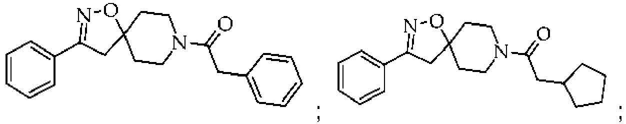

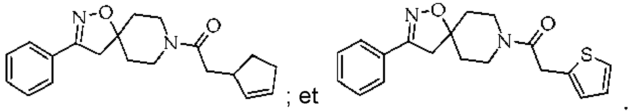

10. Verbindungen nach einem der Ansprüche 1 bis 9 zur Verwendung als Medikament.

11. Verbindungen nach einem der Ansprüche 1 bis 9 zur Verwendung bei der Behandlung von bakteriellen und mykobakteriellen Infektionen.

12. Verbindungen nach einem der Ansprüche 1 bis 9 zur Verwendung bei der Behandlung von Tuberkulose, Lepra oder atypischer Mykobakteriose.

13. Pharmazeutische Zusammensetzung, umfassend als aktiven Bestandteil mindestens eine Verbindung nach einem der Ansprüche 1 bis 9 und einen pharmazeutisch annehmbaren Hilfsstoff.

14. Zusammensetzung nach Anspruch 13, **dadurch gekennzeichnet, dass** sie außerdem als Wirkstoff mindestens ein aktives Antibiotikum umfasst, das unter den Antibiotika ausgewählt ist, die über den EthA-Enzymweg aktivierbar sind.

15. Zusammensetzung nach Anspruch 13, **dadurch gekennzeichnet, dass** sie außerdem als Wirkstoff mindestens ein aktives Antibiotikum umfasst, ausgewählt aus Ethionamid, Prothionamid, Isoxyl und Thiacetazon.

## Claims

1. Spiroisoxazoline compounds of general formula (I): 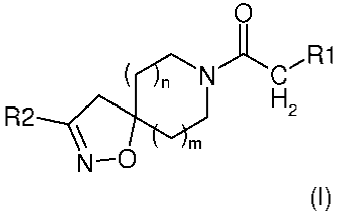 in which:
m = 0 or 1;
n = 0 or 1;
R1 represents a group, chosen from:
linear or branched C1-C5 alkyl chains;
linear or branched C1-C5 alkyl chains, substituted by at least one F;
linear or branched C1-C3 alkyl chains substituted by at least one saturated or unsaturated C3-C6 cyclic group;
saturated or unsaturated C3-C6 cyclic groups;
CN, CH₂CN, CH₂N₃;
Phenyl, phenyl substituted by Cl or F, benzyl, benzyl substituted by Cl or F, -O-phenyl, and thiophenyl;
R2 is chosen from:
phenyl;
benzyl optionally substituted with Cl or F;
naphthalenyl;
phenyl substituted by at least one linear or branched C1-C4 alkyl chain;
phenyl substituted by at least one linear or branched C1-C4 alkyl chain and substituted by at least one F;
phenyl substituted with at least one group chosen from OCH₃, OCF₃, Cl, F, CH₃SO₂ and CF₃; phenyl in which two consecutive carbon atoms are substituted by the same O-CH₂-O group; cyclopropyl, cyclobutyl, cyclopentyl, cyclohexyl; and
aromatic heterocycles with 5 or 6 vertices comprising at least one atom in the cycle chosen from S, N and O.

2. Compounds according to Claim 1, **characterized in that** m = n = 1.

3. Compounds according to Claim 1 or 2, **characterized in that** R1 is chosen from the following groups: -CH₂CF₃, -CF₂CF₃, -(CH₂)₂CF₃, -CH₂-isopropyl, cyclopropyl, cyclobutyl, - cyclopentyl, cyclopentenyl, -CH₂-cyclopropyl, -CH₂-cyclobutyl, -CH₂-cyclopentyl, phenyl substituted by Cl or F, benzyl, benzyl substituted by Cl or F, -O- phenyl, and thiophenyl.

4. Compounds according to any one of Claims 1 to 3, **characterized in that** R1 is chosen from -CH₂CF₃ and -(CH₂)₂CF₃.

5. Compounds according to any one of the preceding Claims, **characterized in that** R2 is phenyl or phenyl substituted in ortho by OCH₃, OCF₃, Cl, F or CF₃.

6. Compounds according to any one of Claims 1 to 4, **characterized in that** R2 is chosen from aromatic heterocycles with 5 or 6 vertices which contain at least one atom chosen from S, N and O atoms adjacent to the atom of the heterocycle linked to carbon in alpha of the nitrogen of the 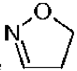 cycle of formula (I).

7. Compounds according to Claim 6, **characterized in that** R2 is chosen from the following groups:

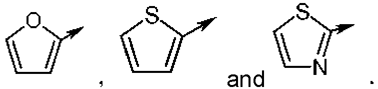

8. Compounds according to any one of Claims 1 to 4, **characterized by** that R2 is chosen from the following groups:

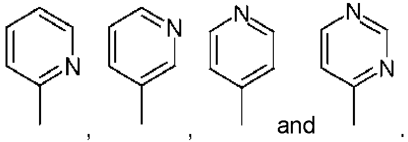

9. Compounds according to Claim 1, **characterized in that** they are chosen from the following compounds:

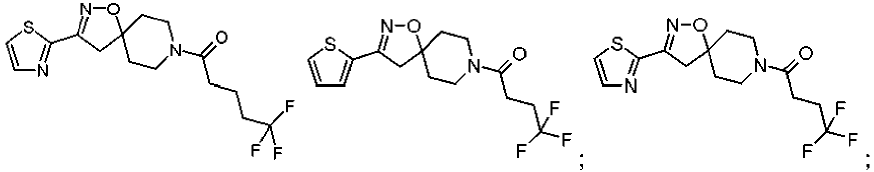

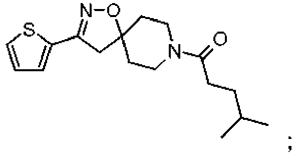

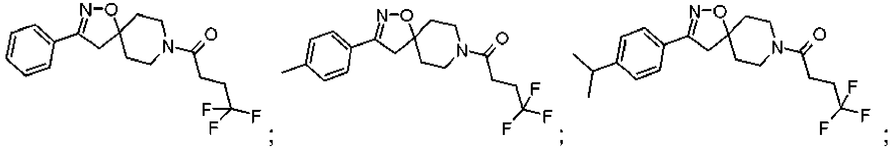

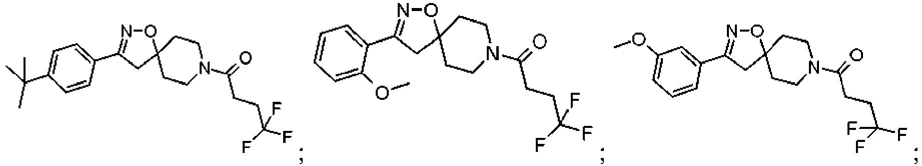

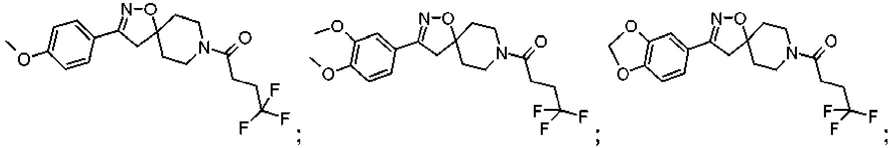

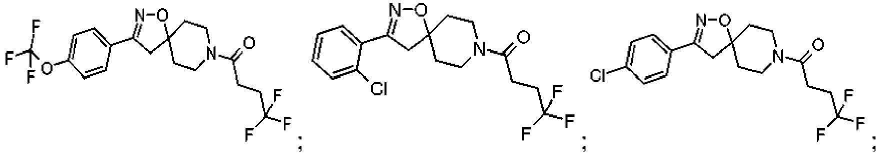

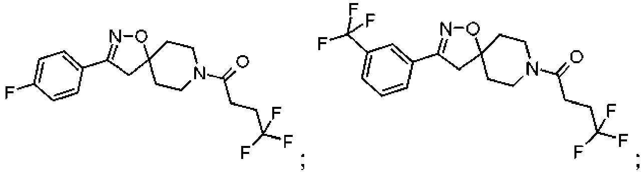

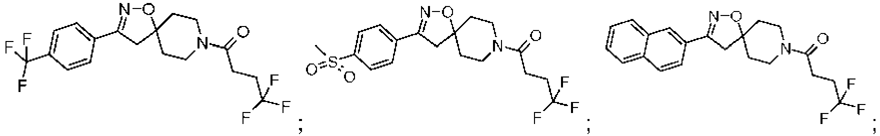

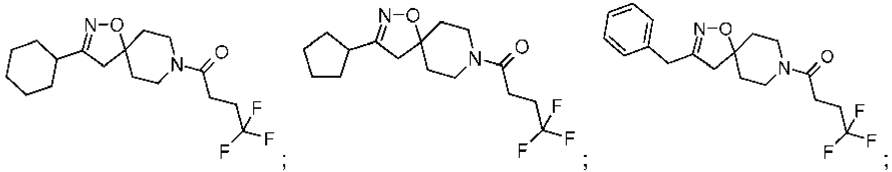

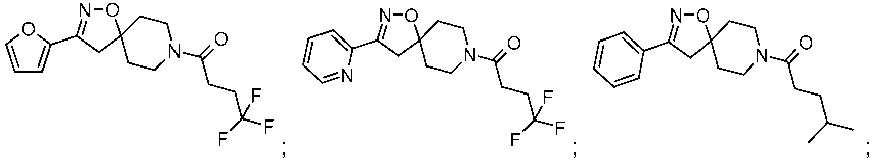

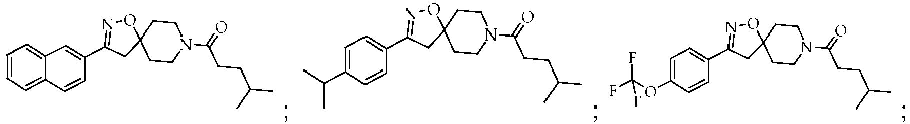

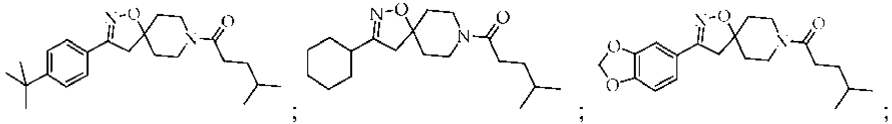

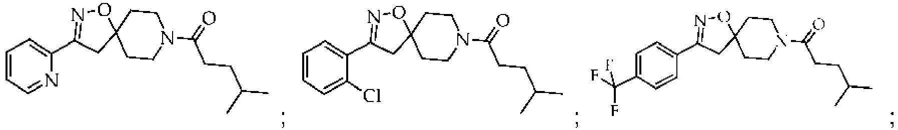

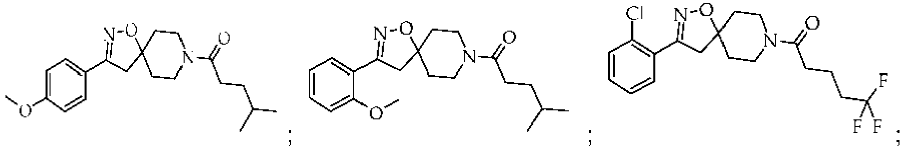

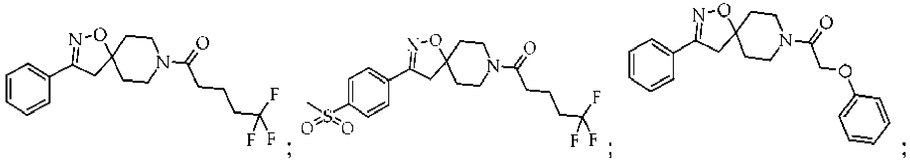

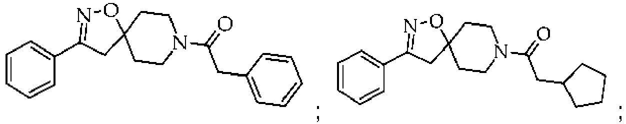

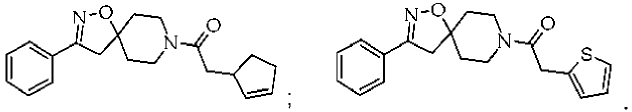

10. Compounds according to any one of Claims 1 to 9 for use as a drug.

11. Compounds according to any of Claims 1 to 9 for use in the treatment of bacterial and mycobacterial infections.

12. Compounds according to any one of Claims 1 to 9 for their use in the treatment of tuberculosis, leprosy or atypical mycobacterioses.

13. Pharmaceutical composition comprising as active principle, at least one compound according to any one of Claims 1 to 9 and a pharmaceutically acceptable excipient.

14. Composition according to Claim 13, **characterized in that** it also includes, as active principle, at least one active antibiotic chosen from antibiotics which can be activated by the EthA enzymatic pathway.

15. Composition according to Claim 13, **characterized in that** it also includes, as active element, at least one active antibiotic, chosen from ethionamide, prothionamide, isoxyl and thiacetazone.
